# FASCICULE DE BREVET EUROPEEN

(11) **EP 0 772 474 B1**
(45) Date de publication et mention de la délivrance du brevet: **20.05.1998**
(21) Numéro de dépôt: 95926422.7
(22) Date de dépôt: 25.07.1995
(51) Int. Cl.: A61N 1/30

(54) **DISPOSITIF D'IONOPHORESE COMPORTANT AU MOINS UN ENSEMBLE ELECTRODE A ELECTRODE COMPOSITE REVERSIBLE**
IONTOPHORESEVORRICHTUNG MIT ZUMINDEST EINER AUS AUSWECHSELBAREN VERBUNDELEKTRODEN BESTEHENDEN ELEKTRODENANORDNUNG
IONTOPHORESIS DEVICE COMPRISING AT LEAST ONE ELECTRODE ASSEMBLY WITH A REVERSIBLE COMPOSITE ELECTRODE

(30) Priorité: 26.07.1994 FR 9409231
(43) Date de publication de la demande: 14.05.1997
(73) Titulaire: ELF AQUITAINE, 92400 Courbevoie (FR); SANOFI, 75008 Paris (FR)
(72) Inventeur: MULLER, Daniel, F-64000 Pau (FR); SAUNAL, Henry, F-34000 Montpellier (FR)
(74) Mandataire: Boillot, Marc
(86) Numéro de dépôt international: FR9500995
(87) Numéro de publication internationale: WO9603179

(56) Documents cités:
- EP-A- 0 556 112

## Description

L'invention concerne un dispositif d'ionophorèse pour l'administration transcutanée d'un principe actif à un sujet, lequel dispositif comporte au moins un ensemble électrode pourvu d'une électrode composite réversible. Elle concerne encore ledit ensemble électrode.

Dans le traitement courant de nombreuses affections, il est nécessaire d'administrer un médicament ou autre principe actif à un sujet de manière contrôlée et souvent prolongée. Parmi les nombreuses techniques mises à la disposition du galéniste, celle de l'ionophorèse représente une alternative intéressante pour le contrôle de l'administration de principes actifs tels que des substances médicamenteuses dans l'organisme du sujet. Une telle technique consiste à utiliser le courant électrique pour contrôler la quantité mais aussi la vitesse de délivrance d'un principe actif à travers la peau d'un sujet. Dans de nombreux cas, cette technique s'avère très efficace en augmentant de manière significative l'apport de principe actif dû au courant, comparativement à la quantité délivrée sans courant.

L'administration transcutanée d'un principe actif par ionophorèse à un sujet est généralement réalisée, à partir d'une solution aqueuse ou d'un gel aqueux renfermant le principe actif sous une forme au moins partiellement ionisée ou sous une forme neutre, en appliquant un signal électrique entre, d'une part, une première électrode, dite électrode active, ayant même polarité que les ions du principe actif à administrer ou une polarité positive si le principe actif est neutre et se trouvant en contact avec un élément réservoir, qui renferme le principe actif et se trouve placé au contact d'une première zone de la peau du sujet, et, d'autre part, une deuxième électrode, dite contre-électrode ou électrode passive, de polarité opposée à celle associée au principe actif, qui est placée, directement ou par le biais d'un électrolyte indifférent, au contact d'une deuxième zone de la peau du sujet distincte de la première zone. Lors du passage du courant, généré par application de la tension entre les électrodes, dans le circuit ainsi réalisé, les ions du principe actif migrent, à l'opposé de l'électrode de même polarité (électrode active), à travers la peau et les tissus du sujet vers l'électrode de polarité opposée (contre-électrode) et se retrouvent ainsi à passer dans le système circulatoire du sujet. De même, les molécules neutres de principe actif sont entraînées, à l'opposé de l'électrode positive dans le flux aqueux d'électro-osmose à travers la peau et les tissus du sujet vers l'électrode négative (contre-électrode) et se retrouvent également ainsi à passer dans le système circulatoire du sujet.

Un dispositif d'ionophorèse pour l'administration transcutanée d'un principe actif à un sujet est du type comportant un premier ensemble électrode constitué d'une première électrode, dite électrode active, en contact avec un élément réservoir actif, adapté, d'une part, pour contenir un électrolyte renfermant le principe actif sous une forme au moins partiellement ionisée ou sous une forme neutre et, d'autre part, pour assurer, lorsqu'il est placé au contact d'une zone de la peau du sujet, un continuum conducteur ionique entre ladite première électrode et ladite zone, un deuxième ensemble électrode constitué soit (i) d'une deuxième électrode, dite contre-électrode, ou bien, de préférence, (ii) d'une telle deuxième électrode en contact avec un élément réservoir agencé pour renfermer un électrolyte et pour assurer, lorsqu'il est placé au contact d'une portion de la peau du sujet, un continuum conducteur ionique entre la deuxième électrode et ladite portion, et un générateur de signaux électriques connectable à chacune desdites première et deuxième électrodes de telle sorte que la première électrode ait même polarité que les ions du principe actif ou une polarité positive si ledit principe actif est neutre et que la deuxième électrode ait une polarité opposée à celle de la première électrode.

Les électrodes d'ionophorèse destinées à appliquer des signaux électriques à des réservoirs médicamenteux, pour favoriser le passage transdermique des principes actifs, doivent répondre aux conditions suivantes :
- bonne conductivité électronique afin que le potentiel appliqué au niveau de la connexion avec le générateur de signaux électriques se répartisse uniformément sur toute la surface du réservoir et donc sur toute la surface de contact avec la peau ;
- réversibilité électrochimique, c'est-à-dire absence de surtension en activité et absence de réactions secondaires avec l'électrolyte, en particulier évitant l'électrolyse de l'eau toujours présente par la nature même du réservoir médicamenteux et/ou par la perspiration, et en outre absence de corrosion.

Depuis de nombreuses années, les électrochimistes utilisent, comme capteurs biologiques ou comme électrodes réversibles pour ionophorèse, des électrodes d'argent/chlorure d'argent (Ag/AgCl), qui sont obtenues notamment par chloruration directe d'un film, d'un fil ou d'une grille d'argent selon des procédés électrochimiques conventionnels d'oxydation anodique dans des solutions aqueuses riches en chlorures, par exemple HCl, NaCl ou autres. Ces électrodes, obtenues par chloruration, donnent satisfaction du point de vue électrochimique, mais elles présentent toutefois les inconvénients suivants :
- la chloruration électrochimique est assez lente et délicate si l'on veut obtenir une bonne qualité de dépôt de chlorure, elle se prête mal à des procédés continus et à la réalisation de zones non chlorurées de formes éventuellement complexes qu'il est souhaitable de ménager pour permettre les prises de contact permanentes avec le générateur de signaux ;
- un excès important de métal argent est nécessaire pour permettre à la fois la chloruration au niveau souhaité, généralement d'environ 0,5 mg/cm² à 16 mg/cm² soit de quoi faire passer une quantité d'électricité de 0,1 à 3 mAh (0,36 à 10,8 Coulombs) par cm² d'électrode, et également une tenue mécanique suffisante de l'électrode ;
- dès que la quantité de chlorure d'argent dépasse une dizaine de mg par cm², ce qui correspond à environ 2 milliampère-heure par cm², l'adhésion du chlorure d'argent sur son support est très mauvaise et ce chlorure d'argent risque de se décoller du support à la moindre sollicitation mécanique rendant l'électrode irréversible ;
- l'utilisation électrochimique de chlorure d'argent formé initialement n'est que partielle car sa réduction, lorsque l'électrode est utilisée comme négative, se fait préférentiellement à l'interface Ag/AgCl, ce qui amoindrit encore la cohésion de l'ensemble et rend cette électrode irréversible bien avant l'épuisement du chlorure d'argent.

Du fait que les électrodes soient jetables à chaque traitement ionophorétique, la notion de coût devient primordiale. Les électrodes à base de films d'argent chloruré sont onéreuses par leur coût matière, qui est dû à un excès d'argent métallique et de chlorure d'argent indispensables pour une bonne reproductibilité, et par leur mode de fabrication discontinu et nécessairement lent, car la chloruration de l'argent doit être conduite à faible densité de courant, généralement inférieure à 5 mA par cm², pour obtenir une bonne qualité de dépôt d'AgCl.

On connaît des ensembles électrodes pour ionophorèse comportant une électrode composite, par exemple à base d'argent ou/et de chlorure d'argent, en contact avec un élément réservoir adapté, d'une part, pour contenir un électrolyte, qui renferme un principe actif sous une forme au moins partiellement ionisée ou sous une forme neutre, si l'électrode associée est l'électrode active, ou bien qui contient un électrolyte indifférent, si l'électrode associée ne joue que le rôle de la contre-électrode, et, d'autre part, pour assurer, lorsqu'il est placé au contact d'une zone de la peau du sujet, un continuum conducteur ionique entre ladite électrode composite et ladite zone. L'électrode composite est formée d'une composition constituée d'un liant polymère et, comptés en volume dudit liant, de 5% à 40% d'une charge conductrice, en particulier noir de carbone ou libres de graphite, formant un réseau conducteur au sein du liant polymère et de 5% à 40% d'une matière divisée consommable par oxydation ou réduction électrochimique. De tels ensembles électrodes et les dispositifs d'ionophorèse qui les renferment sont notamment décrits dans la citation WO-A-9116944. Lorsque l'électrode de l'ensemble électrode est destinée à jouer le rôle d'anode, la matière consommable électrochimiquement se consomme par oxydation et consiste en un métal oxydable électrochimiquement tel que Ag, Zn, Cu, Ni, Sn, Pb, Fe, Cr, les métaux Ag, Zn et Cu étant tout particulièrement préférés. Lorsque l'électrode de l'ensemble électrode est destinée à jouer le rôle de cathode, la matière consommable électrochimiquement se consomme par réduction et consiste généralement en un composé métallique ionisable dont les ions métalliques sont réductibles électrochimiquement. Parmi ces composés métalliques on peut citer avantageusement les halogénures et l'hexacyanoferrate d'argent et les halogénures de cuivre et tout particulièrement AgCl et CuCl. Les polymères susceptibles de constituer le liant polymère de l'électrode composite peuvent être tels que polyalcènes, polyisoprène, polyvinylacétate, copolymères éthylène/acétate de vinyle, polyamides, polyuréthanes, polychlorure de vinyle, polymères cellulosiques, polyoxyéthylène, polymères de l'acide acrylique. Les copolymères préférés sont des copolymères éthylène/acétate de vinyle (en abrégé "EVA").

Par rapport aux électrodes d'ionophorèse constituées d'un film d'une matière consommable électrochimiquement, par exemple, métal tel que cuivre, zinc ou argent ou/et sel métallique tel que CuCl ou AgCl déposé sur un support, les électrodes composites correspondantes telles que décrites dans la citation WO-A-9116944 possèdent des propriétés mécaniques nettement améliorées. Ces électrodes composites présentent une bonne souplesse et l'on n'observe pas de décollement, ni de déchirure de la couche d'électrode composite durant l'utilisation. En outre, la quantité de matière consommable électrochimiquement, à utiliser pour produire l'électrode composite, est plus réduite que celle à utiliser pour constituer l'électrode d'ionophorèse non composite correspondante, car il n'est plus nécessaire d'avoir un excès de la matière consommable électrochimiquement, notamment excès de métal, pour assurer la tenue mécanique de l'électrode, ladite tenue mécanique étant apportée par le liant polymère : il s'ensuit donc une diminution du coût matière.

Malgré leurs avantages sur les électrodes réversibles conventionnelles utilisées en ionophorèse, les électrodes composites proposées dans la citation WO-A-9116944 présentent encore certaines insuffisances. En particulier, il est difficile de réaliser de telles électrodes composites ayant une épaisseur inférieure à 100 µm, c'est-à-dire une capacité inférieure à 2 mA-heure. De plus, ces électrodes composites ne sont quasiment pas inversibles. Par "électrodes inversibles" on entend, selon l'invention, des électrodes utilisables sur plusieurs cycles d'oxydation et de réduction résultant d'un ou plusieurs changements de polarité au cours du traitement ionophorétique. En outre, il est très difficile ou même, cas d'un liant polymère EVA, quasiment impossible d'obtenir lesdites électrodes composites par une technique d'épandage par voie solvant.

On a maintenant trouvé que l'on pouvait remédier aux inconvénients des électrodes composites pour ionophorèse du type de celles décrites dans la citation WO-A-9116944, en constituant le liant polymère desdites électrodes par un polymère particulier renfermant, sous forme polymérisée, de 60% à 100% molaire d'époxy-1,2 propane et/ou d'époxy-1,2 butane et de 40% à 0% molaire d'un ou plusieurs autres monomères copolymérisables avec l'époxy-1,2 propane et/ou l'époxy-1,2 butane.

Lesdites électrodes composites, dont le liant polymère est constitué du polymère particulier précité à base d'époxy-1,2 propane ou/et d'époxy-1,2 butane, outre les avantages des électrodes composites de la citation WO-A-9116944, présentent, entre autres, une excellente inversibilité et, de plus, elles se prêtent bien à une production faisant appel aux techniques d'épandage par voie solvant permettant l'obtention d'électrodes d'épaisseurs inférieures à 100 µm, c'est-à-dire ayant des capacités inférieures à 2 mA-heure.

L'invention a donc pour objet un dispositif perfectionné d'ionophorèse comportant au moins un ensemble électrode équipé d'une électrode composite réversible à liant polymère constitué d'un polymère tel que précité à base d'époxy-1,2 propane ou/et d'époxy-1,2 butane et elle concerne également ledit ensemble électrode.

Le dispositif d'ionophorèse selon l'invention, pour l'administration transcutanée d'un principe actif à un sujet, est du type comportant un premier ensemble électrode constitué d'une première électrode, dite électrode active, en contact avec un élément réservoir actif adapté, d'une part, pour contenir un électrolyte renfermant le principe actif sous une forme au moins partiellement ionisée ou sous une forme neutre et, d'autre part, pour assurer, lorsqu'il est placé au contact d'une zone de la peau du sujet, un continuum conducteur ionique entre ladite première électrode et ladite zone, un deuxième ensemble électrode constitué soit (i) d'une deuxième électrode, dite contre-électrode, ou bien, de préférence, (ii) d'une telle deuxième électrode en contact avec un élément réservoir agencé pour renfermer, au moins, un électrolyte et pour assurer, lorsqu'il est placé au contact d'une portion de la peau du sujet, un continuum conducteur ionique entre la deuxième électrode et ladite portion, et un générateur de signaux électriques connectable à chacune desdites première et deuxième électrodes de telle sorte que la première électrode ait même polarité que les ions du principe actif ou une polarité positive si ledit principe actif est neutre et que la deuxième électrode ait une polarité opposée à celle de la première électrode, la première électrode en contact avec l'élément réservoir actif ou/et la deuxième électrode en contact avec l'élément réservoir lui étant associé consistant en une électrode composite formée d'une composition constituée d'un liant polymère et, en pourcentages volumiques dudit liant, de 4% à 60% d'une charge conductrice pulvérulente ou fibreuse non consommable électrochimiquement et de 4% à 100% d'une matière divisée consommable par oxydation ou réduction électrochimique et il se caractérise en ce que le liant polymère de l'électrode composite ou de chacune des électrodes composites consiste en au moins un polymère à base d'époxy-1,2 propane et/ou d'époxy-1,2 butane et renfermant, en pourcentages molaires, de 60% à 100% d'époxy-1,2 propane ou/et d'époxy-1,2 butane et de 40% à 0% d'un ou plusieurs autres monomères copolymérisables avec l'époxy-1,2 propane ou/et l'époxy-1,2 butane.

L'invention concerne également un ensemble électrode pour ionophorèse comportant une électrode composite en contact avec un élément réservoir adapté, d'une part, pour contenir un électrolyte, ledit électrolyte renfermant un principe actif sous une forme au moins partiellement ionisée ou sous une forme neutre ou étant un électrolyte indifférent, et, d'autre part, pour assurer, lorsqu'il est placé au contact d'une zone de la peau d'un sujet, un continuum conducteur ionique entre ladite électrode composite et ladite zone, laquelle électrode composite est formée d'une composition constituée d'un liant polymère et, comptés en volume dudit liant, de 4% à 60% d'une charge conductrice pulvérulente ou fibreuse non consommable électrochimiquement et de 4% à 100% d'une matière divisée consommable par oxydation ou réduction électrochimique, ledit ensemble se caractérisant en ce que le liant polymère de l'électrode composite consiste en au moins un polymère à base d'époxy-1,2 propane ou/et d'époxy-1,2 butane et renfermant, en pourcentages molaires, de 60% à 100% d'époxy-1,2 propane ou/et d'époxy-1,2 butane et de 40% à 0% d'un ou plusieurs autres monomères copolymérisables avec l'époxy-1,2 propane ou/et l'époxy-1,2 butane.

Les polymères à base d'époxy-1,2 propane (encore appelé oxyde de propylène-1,2) ou/et d'époxy-1,2 butane (encore appelé oxyde de butylène-1,2), parmi lesquels sont choisis le ou les polymères constituant le liant polymère de l'électrode composite, ont plus particulièrement une teneur molaire en époxy-1,2 propane ou/et époxy-1,2 butane allant de 75% à 100%. Lesdits polymères à base d'époxy-1,2 propane ou/et d'époxy-1,2 butane sont plus spécialement des homopolymères de l'époxy-1,2 propane ou de l'époxy-1,2 butane ou encore des copolymères statistiques, séquencés ou séquencés à charnières statistiques de l'époxy-1,2 propane et de l'époxy-1,2 butane entre eux ou/et avec un ou plusieurs monomères cycliques choisis dans le groupe formé de (i) les oxydes cycliques dont le cycle comporte plus de trois maillons et (ii) les oxydes cycliques de formule 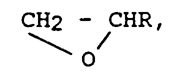 dans laquelle R est un radical R₁ ou un radical CH₂ - 0 - R₂ - R₁, avec R₁ désignant un atome d'hydrogène ou un radical alkyle en C₃ à C₁₂ et de préférence en C₃ à C₆ ou akényle en C₂ à C₁₂ et de préférence en C₂ à C₆ et R₂ représentant un radical polyéther de formule (̵CH₂ - CH₂ - 0)̵ₚ avec p désignant un nombre allant de 0 à 10 et de préférence de 0 à 4.

Parmi les oxydes cycliques (i) à plus de trois maillons dans le cycle, on peut citer l'oxétane, le trétrahydrofurane, le dioxolane, le dioxane et leurs dérivés substitués, notamment par des radicaux alkyles en C₁ à C₄ tels que méthyle ou éthyle. Parmi les oxydes cycliques (ii) de formule 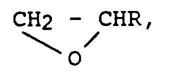 on peut citer ceux pour lesquels R représente un atome d'hydrogène ou un radical propyle, butyle, -CH₂0CH₃, -CH₂0CH₂ - CH = CH₂.

Les polymères à base d'époxy-1,2 propane ou/et d'époxy-1,2 butane, constituant le liant de l'électrode composite, qu'ils soient des homopolymères ou des copolymères tels que définis plus haut, peuvent être réticulés. La réticulation peut résulter, par exemple, de l'action d'un générateur chimique de radicaux libres, notamment peroxyde, azobisnitrile, soufre, polysulfure, de l'action d'un rayonnement ionisant, par exemple rayonnement γ, ou bien encore de l'action d'un triisocyanate ou d'un composé d'un élément polyvalent choisi parmi Al, Zn, Mg, Cd, Sn, B et Ti.

A l'état non réticulé, le ou les polymères à base d'époxy-1,2 propane ou/et d'époxy-1,2 butane utilisés pour former le liant de l'électrode composite, ont des masses moléculaires en nombre comprises entre 10 000 et 10⁶ et plus particulièrement allant de 30 000 à 500 000.

La charge conductrice non consommable électrochimiquement, entrant dans la composition de l'électrode composite, consiste notamment en un matériau conducteur formé d'au moins un produit tel que noir de carbone, graphite ou bore. Ladite charge et notamment ledit matériau se présentent sous la forme d'une poudre ayant avantageusement une granulométrie inférieure à 100 µm et de préférence inférieure à 50 µm ou bien sous la forme de fibres courtes ayant avantageusement un diamètre allant de 1 µm à 15 µm et un rapport de la longueur au diamètre, exprimés dans la même unité de longueur, allant de 50 à 1000 .

La matière consommable électrochimiquement, qui est associée au liant polymère et à la charge conductrice non consommable électrochimiquement, doit être une matière consommable par oxydation électrochimique lorsque l'électrode est destinée à jouer le rôle d'anode ou une matière consommable par réduction électrochimique lorsque l'électrode est destinée à jouer le rôle de cathode. La matière consommable par oxydation électrochimique est avantageusement un métal choisi parmi les métaux oxydables électrochimiquement tels que Ag, Zn, Cu, Ni, Sn, Pb, Fe, Cr, les métaux Ag, Zn et Cu étant préférés. La matière consommable par réduction électrochimique consiste avantageusement en un composé métallique ionisable donnant des ions métalliques réductibles électrochimiquement, ledit composé métallique étant en particulier un halogénure ou un hexacyanoferrate d'argent ou un halogénure de cuivre et tout spécialement AgCl ou CuCl. La matière consommable électrochimiquement peut également consister en le couple constitué d'un composé ionisable d'un métal, donnant des ions métalliques réductibles électrochimiquement en le métal, et dudit métal, comme c'est le cas, par exemple, du couple AgCl/Ag.

Lorsque l'électrode composite ne renferme qu'une matière consommable par oxydation électrochimique, elle ne devient inversible qu'après un premier cycle de fonctionnement en anode. Si la matière consommable de l'électrode composite n'est consommable que par réduction électrochimique, ladite électrode ne devient inversible qu'après un premier cycle de fonctionnement en cathode. Lorsque la matière consommable de l'électrode composite est constituée d'un couple sel métallique/métal, par exemple couple AgCl/Ag, l'électrode peut fonctionner dès le départ indifféremment en anode ou en cathode.

La matière consommable électrochimiquement, contenue dans l'électrode composite, se présente sous la forme d'une poudre dont la granulométrie est avantageusement inférieure à 100 µm et de préférence inférieure à 50 µm. En particulier, la poudre de la matière consommable électrochimiquement présente une distribution polymodale de granulométrie, c'est-à-dire est constituée d'un mélange d'au moins deux poudres de granulométries différentes, chacune desdites granulométries étant dans la plage de granulométrie inférieure à 100 µm et de préférence inférieure à 50 µm.

On peut envisager de déposer la matière consommable électrochimiquement sur la charge conductrice pour former un matériau bifonctionnel combinant la fonction de la matière consommable électrochimiquement et la fonction de la charge conductrice et ensuite d'incorporer ledit matériau bifonctionnel au liant polymère pour constituer l'électrode composite. Ainsi, on pourrait produire des fibres de carbone ou de graphite revêtues d'un métal se consommant par oxydation électrochimique et incorporer les fibres ainsi revêtues au liant polymère pour former l'électrode composite.

Les proportions de charge conductrice non consommable électrochimiquement et de matière consommable électrochimiquement associées au liant polymère pour constituer l'électrode composite représentent, comptées en volume du liant polymère, 4% à 60% et plus spécialement 8% à 45% pour la charge conductrice et 4% à 100% et de préférence 25% à 70% pour la matière consommable électrochimiquement.

Les électrodes composites utilisées selon l'invention peuvent être préparées en faisant appel à toute technique d'obtention de films minces à liant polymère et en particulier en utilisant la technique d'enduction sur film support, technique qui peut être comparée à l'application d'une peinture. On obtient ainsi des électrodes composites plates, souples et minces, bien adaptées à une utilisation en application sur la peau.

Dans ladite technique d'enduction, on prépare tout d'abord une phase fluide renfermant en proportions appropriées, comprises dans les intervalles définis précédemment, le polymère à base d'époxy-1,2 propane ou/et d'époxy-1,2 butane, qui est destiné à constituer la matrice polymère de l'électrode composite, la charge conductrice non consommable électrochimiquement et la matière consommable électrochimiquement, puis on dépose par enduction une couche de ladite phase fluide sur un support, ladite couche, après refroidissement ou/et évaporation des matières volatiles, constituant l'électrode composite.

Lorsque le polymère à base d'époxy-1,2 propane ou/et d'époxy-1,2 butane est suffisamment fluide, aux températures de travail, pour former un mélange fluide avec les autres constituants de l'électrode composite, un tel mélange peut être utilisé pour le dépôt de la couche sur le support par enduction.

Avantageusement, la phase fluide à partir de laquelle on forme la couche d'électrode composite sur le support par enduction, consiste en une dispersion de la charge conductrice et de la matière consommable électrochimiquement dans une solution du polymère à base d'époxy-1,2 propane ou/et d'époxy-1,2 butane dans un solvant dudit polymère. Par cette technique d'enduction par voie solvant, on peut obtenir aisément des électrodes composites dont l'épaisseur est inférieure à 100 µm et, par exemple, comprise entre 10 µm et 100 µm.

Eventuellement, la matrice polymère de l'électrode composite peut être réticulée, lors du dépôt de la couche d'électrode composite sur le support par enduction à partir de la phase fluide. Comme indiqué précédemment, cette réticulation peut être effectuée en incluant un agent de réticulation, par exemple générateur chimique de radicaux libres, triisocyanate, composé d'élément polyvalent comme cité plus haut, dans la phase fluide utilisée pour l'enduction ou en soumettant la couche de phase fluide déposée sur le support à l'action d'un rayonnement ionisant.

Dans une forme de réalisation du dispositif d'ionophorèse selon l'invention, qui permet de réaliser l'administration transcutanée d'une quantité totale donnée d'un principe actif à un sujet, l'électrode de l'un des ensembles électrodes est agencée pour constituer une électrode composite particulière, dite électrode composite limitante, laquelle électrode limitante est une électrode composite selon l'invention qui renferme une quantité limitée de la matière consommable électrochimiquement, ladite quantité limitée étant choisie pour que la quantité d'électricité nécessaire à sa consommation électrochimique corresponde à la quantité d'électricité nécessaire pour administrer la quantité totale donnée de principe actif au sujet, de telle sorte que la circulation du courant entre les électrodes soit pratiquement interrompue lorsque la matière consommable de l'électrode composite limitante a été consommée.

Dans le dispositif d'ionophorèse selon l'invention, un seul des ensembles électrodes, de préférence l'ensemble renfermant le principe actif, peut être équipé d'une électrode composite réversible selon l'invention, l'autre ensemble électrode comportant une électrode réversible non composite ou même une électrode non réversible, par exemple une électrode en carbone, graphite, platine, titane, ou acier inoxydable. Il est cependant préférable que chacun des ensembles électrodes du dispositif d'ionophorèse soit équipé d'une électrode composite selon l'invention, l'un des ensembles électrodes étant muni d'une électrode composite adaptée pour jouer initialement le rôle d'anode, c'est-à-dire ne renfermant initialement qu'une matière consommable par oxydation électrochimique, et l'autre des ensembles électrodes étant pourvu d'une électrode composite adaptée pour jouer initialement le rôle de cathode, c'est-à-dire ne renfermant initialement qu'une matière consommable par réduction électrochimique. On peut également envisager le cas où chacun des ensembles électrodes du dispositif d'ionophorèse est équipé d'une électrode composite selon l'invention renfermant initialement un couple de matières consommables électrochimiquement la première par oxydation et la deuxième par réduction et qui se transforment la première en la deuxième par oxydation électrochimique et la deuxième en la première par réduction électrochimique, un tel couple étant par exemple Ag/AgCl. Dans ce cas, l'électrode de chacun des ensembles électrodes peut jouer initialement le rôle d'anode ou de cathode selon que sa polarité est positive ou négative.

Le générateur électrique applique entre les électrodes du dispositif d'ionophorèse un signal électrique, qui peut être soit un signal intensiométrique, c'est-à-dire un signal d'intensité moyenne imposée, par exemple constante (signal intensiostatique), soit, de préférence', un signal potentiométrique, c'est-à-dire un signal de tension moyenne imposée, par exemple constante (signal potentiostatique). Le signal électrique du type intensiostatique ou du type potentiostatique peut être continu ou pulsé et permanent ou intermittent, avec ou sans inversion temporaire de polarité. La fréquence du signal électrique peut aller de 0 à 500 kHz et plus particulièrement de 0 à 100 kHz.

Avantageusement, la tension moyenne du signal électrique appliqué entre les deux électrodes, à savoir électrode active et contre-électrode, du dispositif d'ionophorèse est choisie entre 0,1 à 50 Volts et plus spécialement entre 0,5 et 20 Volts de telle sorte que la densité du courant moyen généré entre lesdites électrodes ait une valeur inférieure à 5 mA/cm² et plus particulièrement inférieure ou égale à 1 mA/cm².

Le générateur de signaux électriques du dispositif d'ionophorèse peut être de tout type connu permettant de générer des signaux électriques d'intensité moyenne imposée ou de tension moyenne imposée, qui sont continus ou pulsés et permanents ou intermittents, avec ou sans inversion temporaire de polarité, et qui présentent les caractéristiques définies ci-dessus.

L'électrolyte, qui est présent dans l'élément réservoir actif en contact avec l'électrode composite active, contient avantageusement une solution aqueuse ou un gel aqueux, adhésif ou non, qui renferme le principe actif à administrer sous une forme au moins partiellement ionisée ou sous une forme neutre. De même, l'électrolyte indifférent, qui est éventuellement en contact avec la contre-électrode, se présente, au moins en partie, sous la forme d'une solution aqueuse ou d'un gel aqueux adhésif ou non. Ces solutions ou gels aqueux peuvent constituer la totalité de l'électrolyte présent dans l'élément réservoir actif ou la totalité de l'électrolyte indifférent ou bien peuvent former une partie seulement desdits électrolytes et être alors dispersés dans un milieu non aqueux formant le reste de l'électrolyte et choisi pour ne pas interrompre le continuum conducteur ionique entre l'électrode et la peau et pour accroître la qualité de l'adhésion entre l'électrode et la peau. Ces solutions aqueuses ou gels aqueux peuvent être obtenus comme il est bien connu dans les techniques d'ionophorèse. Des exemples de gels aqueux ou de solutions aqueuses épaisses sont notamment décrits respectivement dans les citations US-A-4 764 164 et US-A-3 163 166.

Le milieu aqueux renfermant le principe actif, de même que le milieu aqueux constituant l'électrolyte indifférent, lorsque ledit électrolyte est utilisé, peuvent renfermer, si besoin est, des additifs divers et notamment des agents susceptibles de favoriser le passage transcutané du principe actif comme, par exemple, des agents vasodilatateurs et/ou des agents amphiphiles parmi lesquels on peut citer, à titre non limitatif des composés du type alcool ou du type ester.

L'utilisation d'électrodes composites selon l'invention dans les dispositifs d'ionophorèse permet de contrôler le pH du milieu aqueux renfermant le principe actif, de même que le pH du milieu aqueux constituant l'électrolyte indifférent, ce qui permet d'éviter ou tout au moins de réduire fortement l'introduction d'ions étrangers dans la peau à partir du milieu aqueux renfermant le principe actif car il n'est plus nécessaire d'utiliser des agents tampons.

Le dispositif d'ionophorèse selon l'invention peut être réalisé à partir de tout dispositif d'ionophorèse, que l'on a modifié pour remplacer au moins l'électrode associée au réservoir actif renfermant le principe actif et, de préférence, l'électrode active et la contre-électrode, par une électrode composite selon l'invention. Le cas échéant, une électrode composite du dispositif d'ionophorèse selon l'invention peut être agencée, comme indiqué précédemment, pour constituer une électrode limitante.

En particulier, le dispositif selon l'invention peut être un dispositif autonome portable, à fixer par bracelet ou éventuellement à coller sur la peau, comportant des électrodes, dont au moins l'une est une électrode composite selon l'invention, ayant chacune une aire inférieure à 50 cm² et plus particulièrement comprises entre 1 cm² et 40 cm² ainsi qu'un générateur miniaturisé de signaux électriques. Ainsi, un dispositif portable autonome selon l'invention peut avoir une structure analogue à celle des dispositifs d'ionophorèse portables autonomes décrits, par exemple, dans les citations US-A-4325367, US-A-4557723, EP-A-0060452 et FR-A-2509182 sous réserve qu'au moins l'électrode active associée à l'élément réservoir actif renfermant le principe actif soit une électrode composite à liant polymère selon l'invention, chacune des électrodes dudit dispositif portable autonome, dont l'une peut être agencée pour constituer une électrode limitante, ayant une aire inférieure à 50 cm² et plus particulièrement comprise entre 1 et 40 cm².

Chacune des électrodes composites du dispositif d'ionophorèse peut être perforée dans le sens de l'épaisseur par une pluralité de microcanaux réalisés, par exemple, mécaniquement par perforation de l'électrode à l'issue de sa fabrication ou bien produits en incorporant un agent porogène hydrosoluble à l'électrode lors de la fabrication de cette dernière, comme indiqué dans la citation WO-A-9116944. La présence de ces microcanaux dans l'électrode composite permet de produire à l'état non-hydraté l'élément réservoir associé à l'électrode et d'hydrater ultérieurement ledit élément réservoir par passage d'eau à travers lesdits canaux.

Lorsque les ensembles électrodes du dispositif d'ionophorèse sont fixés à la peau au moyen d'un adhésif, ceci peut être réalisé en munissant d'une couche d'un adhésif conduisant les ions la face, destinée à venir au contact de la peau, de l'élément réservoir de chaque ensemble électrode ou une zone entourant ladite face.

Le dispositif d'ionophorèse selon l'invention permet d'administrer à un sujet, par voie transcutanée, des principes actifs divers et, notamment, des molécules thérapeutiques telles que, par exemple, insuline, métoprolol, hydrocodone, tétracyclines, salbutamol, acide valproïque, propanolol, arginine-desmopressine, despopressine ou autres. Plus généralement, les principes actifs administrables à un sujet, par voie transcutanée, en faisant appel au dispositif d'ionophorèse selon l'invention incluent les divers produits ou composés mentionnés aux pages 15 et 16 de la citation WO-A-9116944.

L'invention est illustrée par les exemples suivants donnés à titre non limitatif.

### EXEMPLE 1

On préparait quatre séries d'électrodes utilisables comme cathodes dans un dispositif d'ionophorèse, à savoir une série d'électrodes composites selon l'invention renfermant un liant consistant en un homopolymère d'époxy-1,2 propane (électrodes de type 1A), deux séries d'électrodes Composites témoins renfermant l'une un liant consistant en un polyisobutène (électrodes de type 1B) et l'autre un liant consistant en un copolymère EVA (électrodes de type 1C) et enfin une série d'électrodes non composites consistant en un film d'argent chloruré (électrodes de type 1D).

La fabrication des électrodes était réalisée comme décrit ci-après.

### ELECTRODES DE TYPE 1A PREPAREES PAR VOIE SOLVANT

Dans la cuve en acier inoxydable de 50 ml d'un broyeur à billes, on introduisait 3,34g de chlorure d'argent en poudre ayant une granulométrie comprise entre 5 µm et 50 µm, 0,335g de noir de carbone, à savoir noir d'acétylène YS commercialisé par SHELL Chimie, et 8,83 g d'une solution d'un homopolymère d'époxy-1,2 propane (poly [oxypropylène-1,2]) à 15% en poids dans l'éthanol, ledit homopolymère ayant une masse moléculaire en nombre d'environ 100 000, ainsi que quelques billes acier ayant un diamètre de 6 à 10mm.

Après une heure de broyage, on obtenait une pâte homogène que l'on épandait sur un film de polyester ayant 80 µm d'épaisseur, en utilisant une râcle calibrée permettant d'épandre une épaisseur humide de 500 µm environ.

Après séchage en étuve ventilée, on obtenait sur le film de polyester un dépôt sec à la fois adhérent et souple résistant bien aux déformations du film support. L'enduit déposé sur le support présentait un grammage de 9,425 mg/m², soit 6,29 mg de chlorure d'argent par cm² susceptible de réduction en argent par passage de 1,18 mAh/cm². Le film revêtu du dépôt à base de la composition d'homopolymère d'époxy-1,2 propane, de chlorure d'argent et de noir de carbone était utilisé pour la fabrication des électrodes de type A.

Pour ce faire, on découpait dans ledit film enduit, c'est-à-dire revêtu du dépôt précité, des rectangles de 5 cm de longueur et 3 cm de largeur. Sur la face enduite de chaque rectangle, on collait un carré d'un film plastique isolant auto-adhésif ayant 3 cm de côté et présentant dans sa partie centrale une ouverture circulaire centrée au centre du carré et ayant un diamètre de 16 mm, c'est-à-dire une surface de 2cm², le collage du carré étant réalisé de telle sorte qu'un côté du carré Coïncidât avec un bord latéral du rectangle. Sur la partie de l'enduit située en dehors de la zone délimitée par le carré, on appliquait un vernis conducteur à base d'argent afin de faciliter la connexion des électrodes avec le générateur de signaux.

### ELECTRODES DE TYPE 1B PREPAREES PAR VOIE SOLVANT :

Dans la cuve en acier inoxydable de 50 ml d'un broyeur à billes, on introduisait 3,442 g de chlorure d'argent ayant la forme d'une poudre de granulométrie comprise en 5 µm et 50 µm, 0,343 g de poudre de graphite ayant une granulométrie comprise entre 0,5 µm et 30 µm et 12,36 g d'une solution de polyisobutène à 35% en poids dans l'hexane, ledit polyisobutène ayant une masse moléculaire en nombre d'environ 150 000, ainsi que quelques billes en acier de diamètre 6 à 10 mm.

Après une heure de broyage, on obtenait une pâte homogène que l'on épandait sur un film de polyester ayant 80 µm d'épaisseur, en utilisant une râcle calibrée permettant d'épandre une épaisseur humide d'environ 300 µm.

Après séchage en étude ventilée, on obtenait sur le Film de polyester un dépôt sec présentant une bonne adhésion au film support et se conformant, sans décollement ni déchirure, aux déformations dudit film support. L'enduit déposé sur le film support présentait un grammage de 10,42 mg/cm², soit 6,95 mg de chlorure d'argent par cm² susceptible de réduction en argent par passage de 1,2 mAh/cm².

A partir du film enduit obtenu, on préparait des électrodes comme indiqué dans le présent exemple pour les électrodes de type 1A.

### ELECTRODES DE TYPE 1C PREPAREES PAR VOIE SOLVANT :

En utilisant un broyeur à billes possédant une cuve en acier inoxydable de 50 ml, on cobroyait pendant 1 heures, dans du xylène, 3,44 g de chlorure d'argent en poudre de granulométrie comprise entre 5 µm et 50 µm et 0,343 g de noir d'acétylène YS. Le mélange obtenu était ensuite ajouté à une solution d'un copolymère éthylène/acétate de vinyle (copolymère EVA) à 10% en poids dans le xylène maintenue à 80°C, ledit copolymère renfermant 8% en poids d'acétate de vinyle et ayant une masse moléculaire en nombre d'environ 70 000, et l'ensemble était homogénéisé à 80°C à l'aide d'une turbine.

La pâte obtenue était épandue à 80°C sur un film de polyester ayant une épaisseur de 80 µm en utilisant une râcle calibrée permettant d'épandre une épaisseur humide d'environ 600 µm.

Après séchage en étuve ventilée, on obtenait sur le film de polyester un revêtement homogène mais très peu adhérent au support dont il se détache si on le courbe. Ledit revêtement ou enduit déposé sur le film support présentait un grammage de 9,93 mg/cm², soit 6,634 mg/cm² de chlorure d'argent par cm² susceptible de réduction en argent par passage de 1,24 mAh/cm^{2.}

A partir du film enduit ainsi réalisé, on préparait des électrodes comme indiqué plus haut pour les électrodes de type 1A.

### ELECTRODES DE TYPE 1D :

On préparait des électrodes non composites ayant la même configuration que les électrodes composites des types 1A,1B ou 1C, en remplaçant le film de polyester enduit utilisé dans la fabrication desdites électrodes composites par un film d'argent chloruré électrochimiquement et dont l'une des faces était masquée par un film plastique adhésif isolant.

Le film d'argent chloruré électrochimiquement présentait une épaisseur de 15 µm et renfermait une quantité de chlorure d'argent correspondant à une quantité d'électricité égale à 1,25 mAh/cm^{2.}

Pour réaliser la chloruration électrochimique du film d'argent, on faisait passer un courant électrique continu entre ledit film d'argent et un barreau de graphite immergés tous les deux dans un bain d'acide chlorhydrique 1N et reliés le premier au pôle positif et le second au pôle négatif d'un générateur du courant électrique continu, en maintenant le passage du courant électrique pendant 15 minutes et avec une densité de courant égale à 5 mA/cm².

Les diverses électrodes obtenues comme indiqué plus haut étaient soumises à des essais d'évaluation de capacité.

Pour évaluer la capacité réelle des électrodes, chaque électrode à étudier était introduite dans une cellule en forme de cuve parallélépipèdique de 10 ml renfermant une solution aqueuse de Nacl à 1% en poids, de manière à être plongée à une profondeur de 2,5 cm dans ladite solution et ainsi à présenter une surface électrochimiquement active égale à 2 cm^{2.} Une électrode complémentaire de structure et de surface similaires à l'électrode à étudier, mais construite, comme indiqué pour l'électrode 1D, à partir d'une feuille d'argent chloruré par oxydation électrochimique pour renfermer une quantité de chlorure d'argent équivalente à 2 mAh/cm², était également plongée dans la solution aqueuse à la même profondeur que l'électrode à étudier. Ladite électrode à étudier et l'électrode complémentaire étaient reliées, chacune, à l'un des pôles d'un générateur de courant électrique continu délivrant un courant électrique d'intensité constante. Un milliampèremètre et un coulomètre étaient montés en série avec le générateur de courant électrique et un millivoltmètre était disposé pour mesurer la différence de potentiel aux bornes de l'électrode à étudier et de l'électrode complémentaire.

Les études de tolérance cutanée des signaux électriques ionophorétiques ont montré qu'il peut être très utile d'inverser plusieurs fois le sens du courant au cours du traitement ionophorétique. En conséquence, la capacité de chaque type d'électrode à étudier a été évaluée successivement en sens direct puis en sens inverse, avec une densité de courant de 0,25 mA/cm².

On donne dans le tableau I, ci-après, les capacités successives observées à chaque cycle de sens direct (fonctionnement en cathode) et inverse (fonctionnement en anode) avant que l'électrode à étudier ne devienne irréversible, c'est-à-dire au moment où elle commence à provoquer une surtension (voisine de 300 mV) accompagnée d'électrolyse de l'eau entraînant un accroissement du pH dans la cuve, ainsi que la durée de chaque cycle de fonctionnement en cathode (cycle cathode) ou en anode (cycle anode), la durée totale de l'échange et la capacité totale échangée.

**TABLEAU I**

| **Nature de l'électrode** | **Type 1A** | **Type 1B** | **Type 1C** | **Type 1D** |
|---|---|---|---|---|
| **Liant** | **POP** ^{**a)**} | **PIB** ^{**b)**} | **EVA** ^{**c)**} | **Aucun** ^{**d)**} |
| Capacité théorique en cathode (mAh/cm²) | 1,18 | 1,3 | 1,24 | 1,25 |
| Durée Théorique à 0,25 mA/cm² (heures) | 4,72 | 5,20 | 4,95 | 5 |
| Durée 1er cycle cathode (heures) | 4,67 | 3,67 | 0,3 | 3,75 |
| Utilisation de la capacité théorique | 98% | 70,5% | 6% | 75% |
| Durée 1er cycle anode (heures) | 3,34 | 0,75 | 0,06 | 2,67 |
| Durée 2ème cycle cathode (heures) | 3,25 | 0,42 | 0,05 | 2 |
| Utilisation de la capacité théorique | 70% | 11% | 1% | 40% |
| Durée 2ème cycle anode (heures) | 2,25 | 0,25 | 0 | 1,42 |
| Durée 3ème cycle cathode (heures) | 2,17 | 0,05 | 0 | 1 |
| Utilisation de la capacité théorique | 46% | 1% | | 20% |
| Durée 3ème cycle anode (heures) | 1,5 | 0 | | 0,75 |
| Durée 4ème cycle cathode (heures) | 1,42 | 0 | | 0,5 |
| Utilisation de la capacité théorique | 30% | | | 10% |
| Durée 4ème cycle anode (heures) | 1 | | | 0,41 |
| Durée totale de l'échange (heures) | 19,60 | 5,14 | 0,41 | 12,50 |
| Capacité totale échangée (mAh) | 4,9 | 1,3 | 0,1 | 3,12 |

| | | | | |
|---|---|---|---|---|
| a) : POP = homopolymère d'époxy-1,2 propane | | | | |
| b) : PIB = polyisobutène | | | | |
| c) : EVA = copolymère éthylène/acétate de vinyle | | | | |
| d) : électrode non composite en argent chloruré (Ag/AgCl) | | | | |

La comparaison des résultats rassemblés dans le tableau I fait ressortir l'intérêt majeur d'un liant selon l'invention, dans le cas présent homopolymère d'époxy-1,2 propane, pour la réalisation des électrodes composites. En effet, les taux d'utilisation de la capacité théorique, elle-même liée aux quantités de chlorure d'argent déposées par unité de surface, sont nettement plus élevés que pour les électrodes composites à liant polyisobutène ou copolymère EVA ou que pour les électrodes non composites en argent chloruré. Il en va de même pour la capacité utilisable en sens inverse, c'est-à-dire l'aptitude à continuer de fonctionner de façon réversible lors de l'inversion de courant, car dans ce cas encore le liant selon l'invention conduit à des électrodes Composites nettement plus performantes que les autres liants tels que polyisobutène ou copolymère EVA. On a observé que ce dernier polymère se prête particulièrement mal à l'élaboration d'électrodes composites par voie solvant, probablement en raison de sa cristallinité qui entraîne sa précipitation lors de l'épandage de la pâte avant que celle-ci ait eu le temps de bien lier les particules de chlorure d'argent.

Il faut encore souligner que si les coûts matière des diverses électrodes composites sont sensiblement les mêmes, il n'en va pas de même pour les électrodes non composites en argent chloruré (électrodes de type 1D) qui nécessitent, pour conserver une tenue mécanique acceptable, que moins de la moitié de l'argent soit chloruré et par conséquent que les films d'argent utilisés aient une épaisseur de 10 µm à 15 µm au minimum, ce qui représente une quantité d'argent de 10 à 25 mg/cm² qui n'intervient pas dans le processus électrochimique, mais triple le coût de ces électrodes par rapport aux électrodes composites, et qui est engagée en pure perte car les électrodes d'ionophorèse sont à usage unique.

### EXEMPLE 2 :

On préparait, par voie solvant, deux séries d'électrodes composites mixtes, c'est-à-dire utilisables indifféremment comme anodes ou comme cathodes, à savoir une série d'électrodes composites selon l'invention renfermant un liant consistant en un copolymère d'époxy-1,2 propane et d'allyl-glycidyl éther (électrodes de type 2A) et une série d'électrodes composites témoins renfermant un liant consistant en polyisobutène (électrodes de type 2B).

La fabrication des électrodes était réalisée comme suit :

### Electrodes de type 2A :

Dans la cuve en acier inoxydable de 50 ml d'un broyeur à billes, on introduisait 25,4 g d'une solution à 15% en poids d'un copolymère d'époxy-1,2 propane et d'allyl-glycidyléther dans l'éthanol, ledit copolymère renfermant 2% molaire d'allyl-glycidylether et présentant une masse moléculaire en nombre d'environ 100 000, ainsi que 0,965 g de noir d'acétylène YS, 5,35 g de poudre de chlorure d'argent de granulométrie comprise entre 5 µm et 50 µm et 8,06 g d'argent en poudre de granulométrie comprise entre 5 µm et 5O µm. On disposait également dans la cuve quelques billes en acier inoxydable de diamètre 6 à 10 mm et plaçait ensuite ladite cuve sous agitation pendant une demi-heure environ.

La suspension obtenue était épandue sur un film de polyester d'épaisseur 80 µm en utilisant une râcle réglée à 300 µm. Le revêtement produit avait l'aspect d'une peinture conduisant après séchage, réalisé en étuve ventilée, à un film noir, souple et homogène d'un dépôt composite adhérant bien au film support de polyester et ayant une épaisseur d'environ 50 µm. Ce dépôt composite possédait un grammage de 12,32 mg/cm², dont une quantité d'argent, utilisable en anode, équivalente à 1,3 mAh/cm² et une quantité de chlorure d'argent, utilisable en cathode, équivalente à 0,65 mAh/cm².

A partir du film de polyester revêtu du dépôt composite, on fabriquait des électrodes ayant la configuration et les dimensions des électrodes du type 1A de l'exemple 1 en effectuant le montage des électrodes comme indiqué dans ledit exemple 1.

### Electrodes de type 2B :

On opérait comme indiqué pour la production des électrodes de type 2A, en remplaçant toutefois la solution de copolymère d'époxy-1,2 propane et d'allylglycidyléther par 11 g d'une solution du polyisobutène utilisé dans l'exemple 1 à 35% en poids dans l'hexane et en réglant à 250 µm la râcle utilisée pour l'épandage sur le film de polyester.

Le revêtement produit conduisait après séchage à un film noir, homogène et souple d'un dépôt composite adhérant au film support de polyester et possédant une épaisseur de 45 µm. Ce dépôt composite possédait un grammage de 14,2 mg/cm², dont une quantité d'argent, utilisable en anode, équivalente à 1,56 mAh/cm² et une quantité de chlorure d'argent, utilisable en cathode, équivalente à 0,78 mAh/cm².

### Cyclage comparatif des deux types d'électrodes :

Dans une cellule analogue à celle utilisée pour l'évaluation des électrodes de l'exemple 1 et donc remplie d'une solution aqueuse de NaCl à 1% en poids, on plongeait deux électrodes de type 2A ou deux électrodes de type 2B, chaque électrode présentant une surface électrochimiquement active de 2 cm², et on connectait les deux électrodes de la cellule à un système imposant entre lesdites électrodes un courant continu de O,5 mA avec inversion du sens du courant toutes les 30 minutes, ce qui correspond au passage, dans chaque sens, d'une quantité de courant de 0,125 mAh/cm² pour chaque électrode. Un capteur immergé au centre de la cellule permettait de mesurer et d'enregistrer le pH du milieu et un millivoltmètre monté aux bornes des électrodes permettait de suivre la différence de potentiel apparaissant aux bornes de la cellule.

Avec une cellule équipée d'électrodes de type A, on ne notait ni variation anormale du pH ni élévation de la différence de potentiel en fin de cycle pendant 8 heures, soit 8 cycles complets et 14 inversions de sens du courant.

Avec une cellule équipée d'électrodes de type B, bien que ces électrodes aient une capacité théorique légèrement supérieure à celle des électrodes de type A, on observait des élévations de la différence de potentiel aux bornes de la cellule avant même le troisième changement de polarité et une décroissance rapide du pH dès le milieu du quatrième cycle, soit avant la fin de la deuxième heure de fonctionnement.

### EXEMPLE 3 :

On préparait, par voie fondue, deux séries d'électrodes composites utilisables comme cathodes dans un dispositif d'ionophorèse, à savoir une série d'électrodes composites selon l'invention renfermant un liant consistant en un homopolymère d'époxy-1,2 propane (électrode de type 3A) et une série d'électrodes composites témoins renfermant un liant consistant en un copolymère EVA (électrodes de type 3C).

La fabrication des électrodes était réalisée comme suit.

### Electrodes de type 3A :

Dans la cuve de 50 ml d'un malaxeur BRABENDER (Brabender Instruments, Inc., South Hachensack, New Jersey, Etat-Unis) maintenue à 120°C, on introduisait 23,5 g d'un homopolymère d'époxy-1,2 propane ayant une masse moléculaire en nombre d'environ 180 000, puis 20,3 g de fibres de carbone non ensimées ayant un diamètre de 8 µm et une longueur moyenne de 6 mm et enfin 62,6 g de chlorure d'argent en poudre de granulométrie comprise entre 5 µm et 50 µm. Le mélange ainsi réalisé était homogénéisé dans le malaxeur pendant 20 minutes, avant d'être extrait dudit malaxeur et découpé en parcelles, qui étaient ensuite moulées entre deux films de polyéthylène téréphtalate en opérant dans une presse hydraulique dont la température était réglée à 120°C et la pression à 2 tonnes.

Après 5 cycles de pression à 2 tonnes entrecoupés de périodes de relaxation, on obtenait un film de produit composite dont l'épaisseur était voisine de 80 µm et le grammage égal à 19,11 mg/cm², la quantité de chlorure d'argent, utilisable en cathode, était équivalente à 2,1 mAh/cm².

A partir du film de produit composite dont on masquait l'une des faces par un film plastique adhésif isolant, on produisait des électrodes ayant la configuration et les dimensions des électrodes du type 1A en effectuant le montage des électrodes comme indiqué dans l'exemple 1.

### Electrodes de type 3C :

On opérait comme indiqué pour la production des électrodes de type 3A, en remplaçant toutefois l'homopolymère d'époxy-1,2 propane par la même quantité du copolymère d'éthylène et d'acétate de vinyle (copolymère EVA) utilisé dans l'exemple 1, en régulant la cuve du malaxeur à 135°C et en réglant à 165°C la température de la presse à mouler.

Après 5 cycles de pression entrecoupés de périodes de relaxation, on obtenait un film de produit composite dont l'épaisseur était voisine de 95 µm et le grammage égal à 22,8 mg/cm², la quantité de chlorure d'argent, utilisable en cathode, étant équivalente à 2,5 mAh/cm².

A partir dudit film composite masqué sur une face par un film plastique adhésif isolant, on produisait des électrodes ayant la configuration et les dimensions des électrodes de type 1A en opérant comme indiqué dans l'exemple 1.

### Comparaison des taux d'utilisation des électrodes en cathodes puis au cyclage

En utilisant la procédure décrite dans l'exemple 1, on évaluait la capacité des électrodes composites de type 3A et de type 3C, utilisées en cathode, par mesure de la quantité maximale de courant que l'on peut faire passer par unité de surface d'électrode avant l'apparition des phénomènes liés à l'électrolyse de l'eau, à savoir élévation de la différence de potentiel aux bornes de la cellule et apparition d'une variation sensible du pH dans la cellule. La capacité de chaque type d'électrode 3A et 3C a été étudiée pour une densité constante de courant égale à 0,3 mA/cm², la surface électrochimiquement active de chaque électrode représentant 2 cm².

On donne dans le tableau II, ci-dessous, les capacités observées après chaque cycle de fonctionnement en cathode, ainsi que la durée maximale observée pour chaque cycle de fonctionnement en cathode ou en anode, la durée totale de l'échange, la capacité totale échangée et le pourcentage de la capacité totale échangée.

**TABLEAU II**

| **Nature de l'électrode** | **Type 3A** | **Type 3C** |
|---|---|---|
| **Liant** | **POP** | **EVA** |
| Capacité théorique en cathode (mAh/cm²⁾ | 2,1 | 2,5 |
| Durée Théorique à 0,3 mA/cm² (heures) | 7 | 8,33 |
| Durée 1er cycle cathode (heures) | 6,92 | 8,18 |
| Utilisation de la capacité théorique | 99% | 98% |
| Durée 1er cycle anode (heures) | 1,73 | 0,60 |
| Durée 2ème cycle cathode (heures) | 1,63 | 0,23 |
| Utilisation de la capacité théorique | 23% | 2,8% |
| Durée 2ème cycle anode (heures) | 0,47 | 0,04 ^{(*)} |
| Durée 3ème cycle cathode (heures) | 0,40 | |
| Utilisation de la capacité théorique | 5,7% | |
| Durée 3ème cycle anode (heures) | 0,13 | |
| Durée 4ème cycle cathode (heures) | 0,10 | |
| Durée totale d'échange (heures) | 11,38 | 9,05 |
| Capacité totale échangée (mAh) | 3,41 | 2,7 |
| % de la capacité totale échangée | 162% | 108% |

| | | |
|---|---|---|
| (*) apparition d'une diminution très rapide du pH dans la cellule | | |

L'examen des résultats rassemblés dans le tableau II montre que si les performances des électrodes de type 3A et de type 3C sont voisines lors de la première utilisation, la cyclabilité des électrodes 3A utilisant un liant selon l'invention (homopolymère d'époxy-1,2 propane) est très nettement supérieure a celle des électrodes de type 3C utilisant un liant selon l'état de la technique (copolymère EVA), ce qui implique que le liant selon l'invention est beaucoup plus performant que le liant selon l'état de la technique.

De plus, si l'on compare les résultats du présent exemple à ceux de l'exemple 1, on constate également que les électrodes composites selon l'invention obtenues par la voie solvant (électrode 1A) sont supérieures, surtout en matière de cyclabilité, aux électrodes composites selon l'invention obtenues par la voie fondue.

Pour une même capacité d'électrode utilisable aussi bien en sens unique que lors de traitements avec inversions fréquentes du sens du courant, les liants polymères selon l'invention pour électrodes composites utilisables en ionophorèse sont très largement supérieurs aux liants polymères, notamment polyisobutène ou copolymères EVA, de l'état de la technique. Cette supériorité est encore plus évidente lorsque lesdites électrodes sont obtenues par voie solvant, technique permettant d'obtenir des électrodes composites de faibles épaisseurs correspondant à des capacités théoriques inférieures à 1,5 mAh/cm².

### EXEMPLE 4 :

On étudiait la diffusion transcutanée de valproate de sodium par ionophorèse, en utilisant des électrodes dont l'une était une électrode composite de type 1A ou de type 1C.

On opérait dans des cellules d'ionophorèse de structure identique. Chaque cellule d'ionophorèse était constituée de trois compartiments cylindriques adjacents coaxiaux de 2 cm² de section transversale, à savoir, dans cet ordre, un compartiment donneur, un compartiment receveur et un compartiment de contre-électrode, ces trois compartiments étant séparés, chacun du suivant et de façon étanche, par un morceau de peau de rat nu (OFA hr/hr) servant de membrane pour l'étude de la diffusion transcutanée. Le compartiment donneur, d'un volume de 0,5 ml, renfermait une solution aqueuse à 10 % en poids de valproate de sodium. Le compartiment receveur, d'un volume de 10 ml, renfermait du sérum physiologique additionné de 500 ppm en poids de NaN₃ et était agité à l'aide d'un barreau magnétique. Le compartiment de contre-électrode, identique au compartiment donneur, renfermait 0,5 ml d'une solution aqueuse de NaCl à 2 % en poids ainsi que 500 ppm en poids de NaN₃. A son extrémité opposée au compartiment receveur, le compartiment donneur était équipé, selon le cas, d'une électrode composite de type 1A ou de type 1C selon l'exemple 1 et présentant une surface active égale à 2 cm². Le compartiment de contre-électrode était équipé d'une électrode non composite de type 1D selon l'exemple 1 et présentant une surface active égale à 2 cm². La face active de chaque électrode était tournée du côté de la membrane en peau de rat.

Les échantillons de peau de rat avaient été débarrassés des tissus sous-cutanés et conservés par congélation à -40°C jusqu'à leur montage dans la cellule d'ionophorèse, faces dermiques tournées vers le compartiment receveur, après un passage de 15 minutes à température ambiante dans du sérum physiologique additionné de 0,05 % en poids de NaN₃.

Pour chacun des essais réalisés, quatre cellules d'ionophorèse identiques étaient mises en route simultanément. La surface effective d'échange était de 2 cm² pour chaque peau.

Un générateur de courant continu dont le pôle négatif était relié à l'électrode composite, permettait d'établir entre les électrodes de chaque cellule un signal électrique d'intensité imposée égale à 0,2 mA.

On poursuivait l'opération d'ionophorèse jusqu'à l'apparition d'une différence de potentiel égale à 0,8 volt aux bornes de la cellule d'ionophorèse et l'on arrêtait alors le passage du courant par ouverture du circuit.

Au bout de durées, comptées à partir du début de l'opération d'ionophorèse, égales respectivement à 1 heure, 12 heures et 24 heures, on prélevait 0,5 ml du liquide contenu dans le compartiemnt receveur de la cellule d'ionophorèse et dosait la quantité de valproate de sodium contenue dans chacun des échantillons. A partir des valeurs obtenues par dosage, on calculait la quantité totale de valproate passée dans le compatiment receveur de la cellule d'ionophorèse au bout desdites durées.

On donne dans le tableau III les résultats obtenus moyennés sur quatre essais.

**TABLEAU III**

| Nature de la cathode | 1A | 1C |
|---|---|---|
| Capacité théorique (mAh/cm2) | 1,18 | 1,24 |
| Durée théorique à 0,1 mA/cm2 (heures) | 11,8 | 12,4 |
| Durée moyenne réelle (heures) | 11,4 | 0,75 |

| Quantité de valproate passée dans le compartiment receveur (mg): | | |
|---|---|---|
| . à 1 heure | 0,2 | 0,16 |
| . à 12 heures | 2,4 | 0,25 |
| . à 24 heures | 2,9 | 0,28 |

L'examen des résultats figurant au tableau III fait clairement ressortir la supériorité des électrodes composites selon l'invention, qui peuvent fonctionner nettement plus longtemps que les électrodes composites de capacités comparables obtenues selon l'état de la technique avant l'apparition des phénomènes indésirables liés à l'électrolyse de l'eau.

## Revendications

1. Dispositif d'ionophorèse pour l'administration transcutanée d'un principe actif à un sujet, comportant un premier ensemble électrode constitué d'une première électrode, dite électrode active, en contact avec un élément réservoir actif adapté, d'une part, pour contenir un électrolyte renfermant le principe actif sous une forme au moins partiellement ionisée ou sous une forme neutre et, d'autre part, pour assurer, lorsqu'il est placé au contact d'une zone de la peau du sujet, un continuum conducteur ionique entre ladite première électrode et ladite zone, un deuxième ensemble électrode constitué soit (i) d'une deuxième électrode, dite contre-électrode, ou bien, de préférence, (ii) d'une telle deuxième électrode en contact avec un élément réservoir agencé pour renfermer au moins un électrolyte et pour assurer, lorsqu'il est placé au contact d'une portion de la peau du sujet, un continuum conducteur ionique entre la deuxième électrode et ladite portion, et un générateur de signaux électriques connectable à chacune desdites première et deuxième électrodes de telle sorte que la première électrode ait même polarité que les ions du principe actif ou une polarité positive si ledit principe actif est neutre et que la deuxième électrode ait une polarité opposée à celle de la première électrode, la première électrode en contact avec l'élément réservoir actif ou/et la deuxième électrode en contact avec l'élément réservoir lui étant associé consistant en une électrode composite constituée d'un liant polymère et, en pourcentages volumiques dudit liant, de 4% à 60% d'une charge conductrice pulvérulente ou fibreuse non consommable électrochimiquement et de 4% à 100% d'une matière divisée consommable par oxydation ou réduction électrochimique et se caractérisant en ce que le liant polymère de l'électrode composite ou de chacune des électrodes composites consiste en au moins un polymère à base d'époxy-1,2 propane ou/et d'époxy-1,2 butane et renfermant, en pourcentages molaires, de 60% à 100% d'époxy-1,2 propane ou/et d'époxy-1,2 butane et de 40% à 0% d'un ou plusieurs autres monomères copolymérisables avec l'époxy-1,2 propane ou/et l'époxy-1,2 butane.

2. Dispositif selon la revendication 1, caractérisé en ce que le ou les polymères constituant le liant polymère de l'électrode composite ont une teneur molaire en époxy-1,2 propane ou/et époxy-1,2 butane allant de 75% à 100%.

3. Dispositif selon la revendication 1 ou 2, caractérisé en ce que le ou les polymères constituant le liant polymère de l'électrode composite sont choisis parmi les homopolymères de l'époxy-1,2 propane ou de l'époxy-1,2 butane et les copolymères statistiques, séquencés ou séquencés à charnières statistiques de l'époxy-1,2 propane et de l'époxy-1,2 butane entre eux ou/et avec un ou plusieurs monomères cycliques choisis dans le groupe formé de (i) les oxydes cycliques dont le cycle comporte plus de trois maillons et (ii) les oxydes cycliques de formule 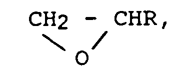 dans laquelle R est un radical R₁ ou un radical -CH2 - 0 - R₂ - R₁, avec R₁ désignant un atome d'hydrogène ou un radical alkyle en C₃ à C₁₂ et de préférence en C₃ à C₆ ou alkényle en C₂ à C₁₂ et de préférence en C₂ à C₆ et R₂ représentant un radical polyéther de formule -(CH₂ - CH₂ - 0)̵ₚ où p désigne un nombre allant de 0 à 10 et de préférence de 0 à 4.

4. Dispositif selon la revendication 3, caractérisé en ce que les monomères cycliques, copolymérisés avec l'époxy-1,2 propane ou/et l'époxy-1,2 butane, sont choisis parmi l'oxétane, le tétrahydrofurane, le dioxolane, le dioxane et les oxydes cycliques de formule 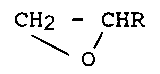 pour lesquels R est un radical propyle, butyle, -CH₂0CH₃ ou -CH₂-0-CH₂ - CH = CH₂.

5. Dispositif selon l'une des revendications 1 à 3, caractérisé en ce que le ou les polymères constituant le liant polymère de l'électrode composite sont des homopolymères de l'époxy-1,2 propane ou de l'époxy-1,2 butane ou des copolymères desdits oxydes entre eux.

6. Dispositif selon l'une des revendications 1 à 5, caractérisé en ce que le liant polymère de l'électrode composite est réticulé.

7. Dispositif selon l'une des revendications 1 à 5, caractérisé en ce que le ou les polymères constituant le liant polymère de l'électrode composite ont des masses moléculaires, à l'état non réticulé, comprises entre 10 000 et 10⁶ et plus particulièrement allant de 30 000 à 500 000.

8. Dispositif selon l'une des revendications 1 à 7, caractérisé en ce que la charge conductrice pulvérulente ou fibreuse non consommable électrochimiquement consiste en un matériau conducteur formé d'au moins un produit tel que noir de carbone, graphite ou bore.

9. Dispositif selon l'une des revendications 1 à 8, caractérisé en ce que la charge conductrice de l'électrode composite est une charge pulvérulente se présentant sous la forme d'une poudre de granulométrie inférieure à 100 µm et de préférence inférieure à 50 µm.

10. Dispositif selon l'une des revendications 1 à 8, caractérisé en ce que la charge conductrice de l'électrode composite est une charge fibreuse se présentant sous la forme de fibres courtes ayant un diamètre allant de 1 µm à 15 µm et un rapport de la longueur au diamètre, exprimés dans la même unité de longueur, allant de 50 à 1000.

11. Dispositif selon l'une des revendications 1 à 10, caractérisé en ce que la matière consommable électrochimiquement présente dans l'électrode composite est consommable par oxydation électrochimique, ladite électrode composite constituant une anode.

12. Dispositif selon la revendication 11, caractérisé en ce que la matière consommable par oxydation électrochimique présente dans l'électrode composite est un métal choisi parmi Ag, Zn, Cu, Ni, Sn, Pb, Fe et Cr, ledit métal étant de préférence Ag, Zn ou Cu.

13. Dispositif selon l'une des revendications 1 à 10, caractérisé en ce que la matière consommable électrochimiquement présente dans l'électrode composite est consommable par réduction électrochimique, ladite électrode composite constituant une cathode.

14. Dispositif selon la revendication 13, caractérisé en ce que la matière consommable par réduction électrochimique présente dans l'électrode composite consiste en AgCl ou CuCl.

15. Dispositif selon l'une des revendications 1 à 10, caractérisé en ce que la matière consommable électrochimiquement présente dans l'électrode composite consiste en le couple constitué d'un composé ionisable d'un métal, donnant des ions métalliques réductibles électrochimiquement en le métal, et dudit métal, ladite électrode constituant indifféremment une anode ou une cathode, lequel couple est tout particulièrement le couple Ag/AgCl.

16. Dispositif selon l'une des revendications 1 à 15, caractérisé en ce que la matière consommable électrochimiquement présente dans l'électrode composite est une poudre, dont la granulométrie est inférieure à 100 µm et de préférence inférieure à 50 µm.

17. Dispositif selon l'une des revendications 1 à 16, caractérisé en ce que les proportions de charge conductrice non consommable électrochimiquement et de matière consommable électrochimiquement associées au liant polymère pour constituer l'électrode composite représentent, comptées en volume du liant polymère, 8% à 45% pour la charge conductrice et 25% à 70% pour la matière consommable électrochimiquement.

18. Dispositif selon l'une des revendications 1 à 17, caractérisé en ce que l'électrode composite ou les électrodes composites qu'il comporte ont une épaisseur inférieure à 100 µm et plus particulièrement comprise entre 10 µm et 100 µm.

19. Dispositif selon l'une des revendications 1 à 18, caractérisé en ce que l'électrode composite ou les électrodes composites qu'il comporte sont produites par une technique d'enduction par voie solvant.

20. Dispositif selon l'une des revendications 1 à 19, caractérisé en ce que le générateur de signaux électriques applique entre les électrodes un signal intensiométrique, c'est-à-dire un signal d'intensité moyenne imposée, ou un signal potentiométrique, c'est-à-dire un signal de tension moyenne imposée, ledit signal étant continu ou pulsé et permanent ou intermittent, avec ou sans inversion temporaire de polarité, et possédant une fréquence allant de 0 à 500 kHz et plus particulièrement de 0 à 100 kHz.

21. Dispositif selon la revendication 20, caractérisé en ce que le signal électrique appliqué entre les électrodes présente une tension moyenne comprise entre 0,1 et 50 Volts et plus spécialement entre 0,5 et 20 Volts de telle sorte que la densité du courant moyen généré entre lesdites électrodes ait une valeur inférieure à 5 mA/cm², et plus particulièrement inférieure ou égale à 1 mA/cm².

22. Ensemble électrode pour ionophorèse comportant une électrode composite en contact avec un élément réservoir adapté, d'une part, pour contenir un électrolyte, ledit électrolyte renfermant un principe actif sous une forme au moins partiellement ionisée ou sous une forme neutre ou étant un électrolyte indifférent, et, d'autre part, pour assurer, lorsqu'il est placé au contact d'une zone de la peau d'un sujet, un continuum conducteur ionique entre ladite électrode composite et ladite zone, laquelle électrode composite est formée d'une composition constituée d'un liant polymère et, comptés en volume dudit liant, de 4% à 60% d'une charge conductrice pulvérulente ou fibreuse non consommable électrochimiquement et de 4% à 100% d'une matière divisée consommable par oxydation ou réduction électrochimique, ledit ensemble se caractérisant en ce que le liant polymère de l'électrode composite consiste en au moins un polymère à base d'époxy-1,2 propane ou/et d'époxy-1,2 butane et renfermant en pourcentages molaires, de 60% à 100% d'époxy-1,2 propane ou/et d'époxy-1,2 butane et de 40% à 0% d'un ou plusieurs autres monomères copolymérisables avec l'époxy-1,2 propane ou/et l'époxy-1,2 butane.

23. Ensemble électrode selon la revendication 22, caractérisé en ce que le ou les polymères constituant le liant polymère de l'électrode composite ont une teneur molaire en époxy-1,2 propane ou/et époxy-1,2 butane allant de 75% à 100%.

24. Ensemble électrode selon la revendication 22 ou 23, caractérisé en ce que le ou les polymères constituant le liant polymère de l'électrode composite sont choisis parmi les homopolymères de l'époxy-1,2 propane ou de l'époxy-1,2 butane et les copolymères statistiques, séquencés ou séquencés à charnières statistiques de l'époxy-1,2 propane et de l'époxy-1,2 butane entre eux ou/et avec un ou plusieurs monomères cycliques choisis dans le groupe formé de (i) les oxydes cycliques dont le cycle comporte plus de trois maillons et (ii) les oxydes cycliques de formule 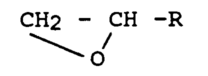 dans laquelle R est un radical R₁ ou un radical -CH₂ -0 - R₂ - R₁, avec R₁ désignant un atome d'hydrogène ou un radical alkyle en C₃ à C₁₂ et de préférence en C₃ à C₆ ou alkényle en C₂ à C₁₂ et de préférence en C₂ à C₆ et R₂ représentant un radical polyéther de formule -(CH₂ -CH₂ - O)̵ₚ où p désigne un nombre allant de 0 à 10 et de préférence de 0 à 4.

25. Ensemble électrode selon la revendication 24, caractérisé en ce que les monomères cycliques, copolymérisés avec l'époxy-1,2 propane ou/et l'époxy-1,2 butane, sont choisis parmi l'oxétane, le tétrahydrofurane, le dioxolane, le dioxane et les oxydes cycliques de formule 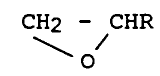 pour lesquels R est un radical propyle, butyle, -CH₂-OCH₃ ou -CH₂-O-CH₂ -CH = CH₂.

26. Ensemble électrode selon l'une des revendications 22 à 24, caractérisé en ce que le ou les polymères constituant le liant polymère de l'électrode composite sont des homopolymères de l'époxy-1,2 propane ou de l'époxy-1,2 butane ou des copolymères desdits oxydes entre eux.

27. Ensemble électrode selon l'une des revendications 22 à 26, caractérisé en ce que le liant polymère de l'électrode composite est réticulé.

28. Ensemble électrode selon l'une des revendications 22 à 26, caractérisé en ce que le ou les polymères constituant le liant polymère de l'électrode composite ont des masses moléculaires, à l'état non réticulé, comprises entre 10 000 et 10⁶ et plus particulièrement allant de 30 000 à 500 000.

29. Ensemble électrode selon l'une des revendications 22 à 28, caractérisé en ce que la charge conductrice pulvérulente ou fibreuse non consommable électrochimiquement consiste en un matériau conducteur formé d'au moins un produit tel que noir de carbone, graphite ou bore.

30. Ensemble électrode selon l'une des revendications 22 à 29, caractérisé en ce que la charge conductrice de l'électrode composite est une charge pulvérulente se présentant sous la forme d'une poudre de granulométrie inférieure à 100 µm et de préférence inférieure à 50 µm.

31. Ensemble électrode selon l'une des revendications 22 à 29, caractérisé en ce que la charge conductrice de l'électrode composite est une charge fibreuse se présentant sous la forme de fibres courtes ayant un diamètre allant de 1 µm à 15 µm et un rapport de la longueur au diamètre, exprimés dans la même unité de longueur, allant de 50 à 1000.

32. Ensemble électrode selon l'une des revendications 22 à 31, caractérisé en ce que la matière consommable électrochimiquement présente dans l'électrode composite est consommable par oxydation électrochimique, ladite électrode composite constituant une anode, ladite matière consommable par oxydation électrochimique étant plus particulièrement un métal choisi parmi Ag, Zn, Cu, Ni, Sn, Pb, Fe et Cr, ledit métal étant de préférence Ag, Zn ou Cu.

33. Ensemble électrode selon l'une des revendications 22 à 31, caractérisé en ce que la matière consommable électrochimiquement présente dans l'électrode composite est consommable par réduction électrochimique, ladite électrode composite constituant une cathode, ladite matière consommable par réduction électrochimique consistant, en particulier, en AgCl ou CuCl.

34. Ensemble électrode selon l'une des revendications 22 à 31, caractérisé en ce que la matière consommable électrochimiquement présente dans l'électrode composite consiste en le couple constitué d'un composé ionisable d'un métal, donnant des ions métalliques réductibles électrochimiquement en le métal, et dudit métal, ladite électrode constituant indifféremment une anode ou une cathode, lequel couple est tout particulièrement le couple Ag/AgCl.

35. Ensemble électrode selon l'une des revendications 22 à 34, caractérisé en ce que la matière consommable électrochimiquement présente dans l'électrode composite est une poudre, dont la granulométrie est inférieure à 100 µm et de préférence inférieure à 50 µm.

36. Ensemble électrode selon l'une des revendications 22 à 35, caractérisé en ce que les proportions de charge conductrice non consommable électrochimiquement et de matière consommable électrochimiquement associées au liant polymère pour constituer l'électrode composite représentent, comptées en volume du liant polymère, 8% à 45% pour la charge conductrice et 25% à 70% pour la matière consommable électrochimiquement.

37. Ensemble électrode selon l'une des revendications 22 à 36, caractérisé en ce que l'électrode composite qu'il comporte possède une épaisseur inférieure à 100 µm et plus particulièrement comprise entre 10 µm et 100 µm.

38. Ensemble électrode selon l'une des revendications 22 à 37, caractérisé en ce que l'électrode composite qu'il comporte est produite par une technique d'enduction par voie solvant.

## Claims

1. Iontophoresis device for the transcutaneous administration of an active ingredient to a subject, comprising a first electrode assembly constituted by a first electrode, known as the active electrode, in contact with an active reservoir element designed, on the one hand, to contain an electrolyte containing the active ingredient in an at least partially ionised form or in a neutral form and, on the other hand, to ensure, when it is placed in contact with a region of the subject's skin, an ion-conductive continuum between the first electrode and said region, a second electrode assembly constituted either (i) by a second electrode, known as the counter-electrode, or preferably (ii) by such a second electrode in contact with a reservoir element designed to contain at least one electrolyte and to ensure, when it is placed in contact with a portion of the subject's skin, an ion-conductive continuum between the second electrode and said portion, and a generator of electrical signals connectable to each of the first and second electrodes in such a manner that the first electrode has the same polarity as the ions of the active ingredient or a positive polarity if the active ingredient is neutral and that the second electrode has an opposite polarity to that of the first electrode, the first electrode in contact with the active reservoir element and/or the second electrode in contact with the reservoir element associated therewith comprising a composite electrode constituted by a polymeric binder and, in percentages by volume of the binder, from 4% to 60% of a non-electrochemically consumable pulverulent or fibrous conductive filler and from 4% to 100% of a divided material consumable by electrochemical oxidation or reduction and being characterised in that the polymeric binder of the composite electrode or of each of the composite electrodes comprises at least one polymer based on 1,2-epoxypropane and/or 1,2-epoxybutane and containing, in molar percentages, from 60% to 100% of 1,2-epoxypropane and/or 1,2-epoxybutane and from 40% to 0% of one or more other monomers copolymerisable with 1,2-epoxypropane and/or 1,2-epoxybutane.

2. Device according to Claim 1, characterised in that the polymer(s) constituting the polymeric binder of the composite electrode has(have) a molar content of 1,2-epoxypropane and/or 1,2-epoxybutane of from 75% to 100%.

3. Device according to Claim 1 or 2, characterised in that the polymer(s) constituting the polymeric binder of the composite electrode is(are) selected from the homopolymers of 1,2-epoxypropane or of 1,2-epoxybutane and the random or block copolymers, or block copolymers having random hinges, of 1,2-epoxypropane and 1,2-epoxybutane with one another and/or with one or more cyclic monomers selected from the group formed by (i) cyclic oxides of which the ring comprises more than three bonds and (ii) cyclic oxides of the formula 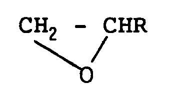 wherein R is a radical R₁ or a radical -CH₂-O-R₂-R₁, in which R₁ represents a hydrogen atom or a C₃ to C₁₂ and preferably C₃ to C₆ alkyl radical or a C₂ to C₁₂ and preferably C₂ to C₆ alkenyl radical and R₂ represents a polyether radical of the formula -(CH₂ - CH₂ - O)̵ₚ where p represents a number from 0 to 10 and preferably from 0 to 4.

4. Device according to Claim 3, characterised in that the cyclic monomers, copolymerised with 1,2-epoxypropane and/or 1,2-epoxybutane, are selected from oxetane, tetrahydrofuran, dioxolane, dioxan and the cyclic oxides of formula 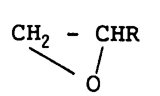 in which R is a propyl, butyl, -CH₂OCH₃ or -CH₂-O-CH₂ - CH = CH₂ radical.

5. Device according to any one of Claims 1 to 3, characterised in that the polymer(s) constituting the polymeric binder of the composite electrode is(are) homopolymers of 1,2-epoxypropane or 1,2-epoxybutane or copolymers of said oxides with one another.

6. Device according to any one of Claims 1 to 5, characterised in that the polymeric binder of the composite electrode is cross-linked.

7. Device according to any one of Claims 1 to 5, characterised in that the polymer(s) constituting the polymeric binder of the composite electrode has(have) molecular masses, in the non-cross-linked state, of between 10,000 and 10⁶ and more especially from 30,000 to 500,000.

8. Device according to any one of Claims 1 to 7, characterised in that the non-electrochemically consumable pulverulent or fibrous conductive filler comprises a conductive material formed by at least one product such as carbon black, graphite or boron.

9. Device according to any one of Claims 1 to 8, characterised in that the conductive filler of the composite electrode is a pulverulent filler in the form of a powder having a granulometry lower than 100 µm and preferably lower than 50 µm.

10. Device according to any one of Claims 1 to 8, characterised in that the conductive filler of the composite electrode is a fibrous filler in the form of short fibres having a diameter of from 1 µm to 15 µm and a ratio of the length to the diameter, expressed in the same unit of length, of from 50 to 1,000.

11. Device according to any one of Claims 1 to 10, characterised in that the electrochemically consumable material present in the composite electrode is consumable by electrochemical oxidation, the composite electrode constituting an anode.

12. Device according to Claim 11, characterised in that the material consumable by electrochemical oxidation and present in the composite electrode is a metal selected from Ag, Zn, Cu, Ni, Sn, Pb, Fe and Cr, the metal preferably being Ag, Zn or Cu.

13. Device according to any one of Claims 1 to 10, characterised in that the electrochemically consumable material present in the composite electrode is consumable by electrochemical reduction, the composite electrode constituting a cathode.

14. Device according to Claim 13, characterised in that the material consumable by electrochemical reduction and present in the composite electrode comprises AgCl or CuCl.

15. Device according to any one of Claims 1 to 10, characterised in that the electrochemically consumable material present in the composite electrode comprises the pair constituted by an ionisable compound of a metal, giving metal ions which can be reduced electrochemically to form the metal, and by said metal, the electrode constituting either an anode or a cathode, which pair is very especially the pair Ag/AgCl.

16. Device according to any one of Claims 1 to 15, characterised in that the electrochemically consumable material present in the composite electrode is a powder of which the granulometry is lower than 100 µm and preferably lower than 50 µm.

17. Device according to any one of Claims 1 to 16, characterised in that the proportions of non-electrochemically consumable conductive filler and of electrochemically consumable material associated with the polymeric binder to constitute the composite electrode represent, in terms of the volume of the polymeric binder, from 8% to 45% in the case of the conductive filler and from 25% to 70% in the case of the electrochemically consumable material.

18. Device according to any one of Claims 1 to 17, characterised in that the composite electrode or the composite electrodes which it comprises has(have) a thickness of less than 100 µm and more especially of between 10 µm and 100 µm.

19. Device according to any one of Claims 1 to 18, characterised in that the composite electrode or the composite electrodes which it comprises is(are) produced by a technique of coating using a solvent.

20. Device according to any one of Claims 1 to 19, characterised in that the generator of electrical signals applies between the electrodes an intensiometric signal, that is to say, a signal of set average intensity, or a potentiometric signal, that is to say, a signal of set average voltage, the signal being continuous or pulsed and permanent or intermittent, with or without temporary inversion of polarity, and having a frequency of from 0 to 500 kHz and more especially from 0 to 100 kHz.

21. Device according to Claim 20, characterised in that the electrical signal applied between the electrodes has an average voltage of between 0.1 and 50 volts and more especially between 0.5 and 20 volts, so that the density of the average current generated between the electrodes has a value lower than 5 mA/cm², and more especially lower than or equal to 1 mA/cm².

22. Electrode assembly for iontophoresis comprising a composite electrode in contact with a reservoir element designed, on the one hand, to contain an electrolyte, the electrolyte containing an active ingredient in an at least partially ionised form or in a neutral form or being an inert electrolyte, and, on the other hand, to ensure, when it is placed in contact with a region of the subject's skin, an ion-conductive continuum between the composite electrode and said region, which composite electrode is formed by a composition constituted by a polymeric binder and, in terms of the volume of the binder, from 4% to 60% of a non-electrochemically consumable pulverulent or fibrous conductive filler and from 4% to 100% of a divided material consumable by electrochemical oxidation or reduction, the assembly being characterised in that the polymeric binder of the composite electrode comprises at least one polymer based on 1,2-epoxypropane and/or 1,2-epoxybutane and containing, in molar percentages, from 60% to 100% of 1,2-epoxypropane and/or 1,2-epoxybutane and from 40% to 0% of one or more other monomers copolymerisable with 1,2-epoxypropane and/or 1,2-epoxybutane.

23. Electrode assembly according to Claim 22, characterised in that the polymer(s) constituting the polymeric binder of the composite electrode has(have) a molar content of 1,2-epoxypropane and/or 1,2-epoxybutane of from 75% to 100%.

24. Electrode assembly according to Claim 22 or 23, characterised in that the polymer(s) constituting the polymeric binder of the composite electrode is(are) selected from the homopolymers of 1,2-epoxypropane or of 1,2-epoxybutane and the random or block copolymers, or block copolymers having random hinges, of 1,2-epoxypropane and 1,2-epoxybutane with one another and/or with one or more cyclic monomers selected from the group formed by (i) cyclic oxides of which the ring comprises more than three bonds and (ii) cyclic oxides of the formula 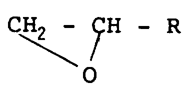 wherein R is a radical R₁ or a radical -CH₂-O-R₂-R₁, in which R₁ represents a hydrogen atom or a C₃ to C₁₂ and preferably C₃ to C₆ alkyl radical or a C₂ to C₁₂ and preferably C₂ to C₆ alkenyl radical and R₂ represents a polyether radical of the formula -(CH₂ - CH₂ - O)̵ₚ where p represents a number from 0 to 10 and preferably from 0 to 4.

25. Electrode assembly according to Claim 24, characterised in that the cyclic monomers, copolymerised with 1,2-epoxypropane and/or 1,2-epoxybutane, are selected from oxetane, tetrahydrofuran, dioxolane, dioxan and the cyclic oxides of formula 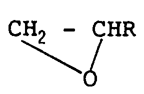 in which R is a propyl, butyl, -CH₂-OCH₃ or -CH₂-O-CH₂ - CH = CH₂ radical.

26. Electrode assembly according to any one of Claims 22 to 24, characterised in that the polymer(s) constituting the polymeric binder of the composite electrode is(are) homopolymers of 1,2-epoxypropane or 1,2-epoxybutane or copolymers of said oxides with one another.

27. Electrode assembly according to any one of Claims 22 to 26, characterised in that the polymeric binder of the composite electrode is cross-linked.

28. Electrode assembly according to any one of Claims 22 to 26, characterised in that the polymer(s) constituting the polymeric binder of the composite electrode has(have) molecular masses, in the non-cross-linked state, of between 10,000 and 10⁶ and more especially from 30,000 to 500,000.

29. Electrode assembly according to any one of Claims 22 to 28, characterised in that the non-electrochemically consumable pulverulent or fibrous conductive filler comprises a conductive material formed by at least one product such as carbon black, graphite or boron.

30. Electrode assembly according to any one of Claims 22 to 29, characterised in that the conductive filler of the composite electrode is a pulverulent filler in the form of a powder having a granulometry lower than 100 µm and preferably lower than 50 µm.

31. Electrode assembly according to any one of Claims 22 to 29, characterised in that the conductive filler of the composite electrode is a fibrous filler in the form of short fibres having a diameter of from 1 µm to 15 µm and a ratio of the length to the diameter, expressed in the same unit of length, of from 50 to 1,000.

32. Electrode assembly according to any one of Claims 22 to 31, characterised in that the electrochemically consumable material present in the composite electrode is consumable by electrochemical oxidation, the composite electrode constituting an anode, and the material consumable by electrochemical oxidation being more especially a metal selected from Ag, Zn, Cu, Ni, Sn, Pb, Fe and Cr, the metal preferably being Ag, Zn or Cu.

33. Electrode assembly according to any one of Claims 22 to 31, characterised in that the electrochemically consumable material present in the composite electrode is consumable by electrochemical reduction, the composite electrode constituting a cathode, and the material consumable by electrochemical reduction comprising especially AgCl or CuCl.

34. Electrode assembly according to any one of Claims 22 to 31, characterised in that the electrochemically consumable material present in the composite electrode comprises the pair constituted by an ionisable compound of a metal, giving metal ions which can be reduced electrochemically to form the metal, and by said metal, the electrode constituting either an anode or a cathode, which pair is very especially the pair Ag/AgCl.

35. Electrode assembly according to any one of Claims 22 to 34, characterised in that the electrochemically consumable material present in the composite electrode is a powder of which the granulometry is lower than 100 µm and preferably lower than 50 µm.

36. Electrode assembly according to any one of Claims 22 to 35, characterised in that the proportions of non-electrochemically consumable conductive filler and of electrochemically consumable material associated with the polymeric binder to constitute the composite electrode represent, in terms of the volume of the polymeric binder, from 8% to 45% in the case of the conductive filler and from 25% to 70% in the case of the electrochemically consumable material.

37. Electrode assembly according to any one of Claims 22 to 36, characterised in that the composite electrode which it comprises has a thickness of less than 100 µm and more especially of between 10 µm and 100 µm.

38. Electrode assembly according to any one of Claims 22 to 37, characterised in that the composite electrode which it comprises is produced by a technique of coating using a solvent.

## Patentansprüche

1. Ionophoresevorrichtung zu transkutanen Verabreichung eines Wirkstoffs an ein Lebewesen, die eine erste Elektrodeneinheit, die aus einer ersten Elektrode, der sogenannten aktiven Elektrode, im Kontakt mit einem aktiven Behälter besteht, der einerseits geeignet ist, einen Elektrolyten aufzunehmen, welcher den Wirkstoff in wenigstens teilweise ionisierter Form oder in neutraler Form enthält, und andererseits so beschaffen ist, daß beim Kontakt mit einer Hautzone des Patienten zwischen der ersten Elektrode und der Zone ein ionisches Leiterkontinuum gebildet wird, eine zweite Elektrodeneinheit, die entweder (i) aus einer zweiten Elektrode, der sogenannten Gegenelektrode, oder vorzugsweise (ii) aus einer solchen zweiten Elektrode im Kontakt mit einem Behälter besteht, der so beschaffen ist, daß er wenigstens einen Elektrolyten enthält und beim Kontakt mit einem Teil der Haut des Lebewesens ein ionisches Leiterkontinuum zwischen der zweiten Elektrode und dem Hautanteil gewährleistet, und eine elektrische Signalerzeugungseinrichtung umfaßt, die sowohl mit der ersten als auch mit der zweiten Elektrode so verbunden werden kann, daß die erste Elektrode dieselbe Polarität wie die Ionen des Wirkstoffs oder eine positive Polarität aufweist, wenn der Wirkstoff neutral ist, und die zweite Elektrode eine der Polarität der ersten Eletkrode entgegengesetzte Polarität aufweist, wobei mindestens die mit dem aktiven Behälter in Berührung stehende erste Elektrode oder die mit dem entsprechenden Behälter in Berührung stehende zweite Elektrode aus einer Verbundstoffelektrode besteht, die aus einem Polymerbindemittel, 4 bis 60% einer elektrochemisch nicht zersetzbaren pulver- oder faserförmigen leitenden Füllung und 4 bis 100%, jeweils bezogen auf das Volumen des Bindemittels, eines durch elektrochemische Oxidation oder Reduktion zersetzbaren feinverteilten Stoffes besteht, und dadurch gekennzeichnet ist, daß das polymere Bindemittel der Verbundstoffelektrode oder jeder der Verbunstoffelektroden aus Wenigstens einem Polymer auf der Basis von 1,2-Epoxy-propan und/oder 1,2-Epoxy-butan besteht und 60 bis 100 Mol.-% 1,2-Epoxy-propan und/oder 1,2-Epoxy-butan und 40 bis 0 Mol.-% eines oder mehrerer anderer mit 1,2-Epoxy-propan und/oder 1,2-Epoxy-butan copolymerisierbarer Monomere enthält.

2. Vorrichtung nach Anspruch 1, dadurch gekennzeichnet, daß das oder die das polymere Bindemittel der Verbundstoffelektrode bildende(n) Polymer(e) einen Molgehalt an 1,2-Epoxy-propan und/oder 1,2-Epoxy-butan von 75 bis 100 % aufweist (aufweisen).

3. Vorrichtung nach Anspruch 1 oder 2, dadurch gekennzeichnet, daß das oder die das polymere Bindemittel der Verbundstoffelektrode bildende(n) Polymer(e) unter Homopolymeren von 1,2-Epoxy-propan oder 1,2-Epoxy-butan und statistischen Copolymeren, Blockcopolymeren oder Blockcopolymeren mit statistischen Gelenken aus 1,2-Epoxy-propan und 1,2-Epoxy-butan zwischen ihnen und/oder mit einem oder mehreren cyclischen Monomeren, ausgewählt aus der Gruppe, bestehend aus (i) cyclischen Oxiden mit einem Ring mit mehr als drei Gliedern und (ii) cyclischen Oxiden der Formel 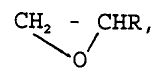 worin R einen Rest R₁ oder einen Rest -CH₂ - O - R₂ - R₁ bedeutet, wobei R₁ ein Wasserstoffatom oder einen C₃₋₁₂- und vorzugsweise C₃₋₆-Alkylrest oder einen C₂₋₁₂- und vorzugsweise C₂₋₆-Alkenylrest bezeichnet und R₂ einen Polyetherrest der Formel -(CH₂-CH₂-O)̵ₚ bedeutet, worin p eine Zahl von 0 bis 10 und vorzugsweise von 0 bis 4 bezeichnet, ausgewählt ist (sind).

4. Vorrichtung nach Anspruch 3, dadurch gekennzeichnet, daß die mit dem 1,2-Epoxy-propan und/oder 1,2-Epoxy-butan copolymerisierten Cyclischen Monomere unter Oxethan, Tetrahydrofuran, Dioxolan, Dioxan und cyclischen Oxiden der Formel 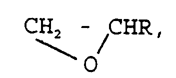 worin R einen Propyl- oder Butylrest, -CH₂OCH₃ oder -CH₂-O-CH₂-CH=CH₂ bedeutet, ausgewählt sind.

5. Vorrichtung nach einem der Ansprüche 1 bis 3, dadurch gekennzeichnet, daß das oder die das polymere Bindemittel der Verbundstoffelektrode bildende(n) Polymer(e) Homopolymere von 1,2-Epoxy-propan oder 1,2-Epoxy-butan oder Copolymere dieser Oxide untereinander sind.

6. Vorrichtung nach einem der Ansprüche 1 bis 5, dadurch gekennzeichnet, daß das polymere Bindemittel der Verbundstoffelektrode vernetzt ist.

7. Vorrichtung nach einem der Ansprüche 1 bis 5, dadurch gekennzeichnet, daß das oder die das polymere Bindemittel der Verbundstoffelektrode bildende(n) Polymer(e) im nichtvernetzten Zustand Molekularmassen von 10.000 bis 10⁶ und insbesondere von 30.000 bis 500.000 aufweist (aufweisen).

8. Vorrichtung nach einem der Ansprüche 1 bis 7, dadurch gekennzeichnet, daß die elektrochemisch nicht zersetzbare pulver- oder faserförmige leitende Füllung aus einem aus wenigstens einem Stoff wie Ruß , Graphit oder Bor gebildeten Material besteht.

9. Vorrichtung nach einem der Ansprüche 1 bis 8, dadurch gekennzeichnet, daß die leitende Füllung der Verbundstoffelektrode eine pulverförmige, in Form eines Pulvers mit einer Korngröße von unter 100 µm und vorzugsweise unter 50 µm vorliegende Füllung ist.

10. Vorrichtung nach einem der Ansprüche 1 bis 8, dadurch gekennzeichnet, daß die leitende Füllung der Verbundstoffelektrode eine faserförmige, in Form von kurzen Fasern mit einem Durchmesser von 1 bis 15 µm und einem Verhältnis Länge : Durchmesser, ausgedrückt in derselben Längeneinheit, von 50 bis 1000 vorliegende Füllung ist.

11. Vorrichtung nach einem der Ansprüche 1 bis 10, dadurch gekennzeichnet, daß der elektrochemisch zersetzbare Stoff in der Verbundstoffelektrode durch elektrochemische Oxidation zersetzbar ist, wobei die Verbundstoffelektrode eine Anode bildet.

12. Vorrichtung nach Anspruch 11, dadurch gekennzeichnet, daß der durch elektrochemische Oxidation zersetzbare Stoff in der Verbundstoffelektrode ein Metall, ausgewählt unter Ag, Zn, Cu, Ni, Sn, Pb, Fe und Cr ist, wobei das Metall vorzugsweise Ag, Zn oder Cu ist.

13. Vorrichtung nach einem der Ansprüche 1 bis 10, dadurch gekennzeichnet, daß der elektrochemisch zersetzbare Stoff in der Verbundstoffelektrode durch elektrochemische Reduktion zersetzbar ist, wobei die Verbundstoffelektrode eine Kathode bildet.

14. Vorrichtung nach Anspruch 13, dadurch gekennzeichnet, daß der durch elektrochemische Reduktion zersetzbare Stoff in der Verbundstoffelektrode aus AgCl oder CuCl besteht.

15. Vorrichtung nach einem der Ansprüche 1 bis 10, dadurch gekennzeichnet, daß der elektrochemisch zersetzbare Stoff in der Verbundstoffelektrode aus einem Paar besteht, das seinerseits aus der ionisierbaren Verbindung eines Metalls, die elektrochemisch zum Metall reduzierbare Metallionen liefert, und dem Metall besteht, wobei die Elektrode gleichermaßen eine Anode oder Kathode bildet, und das Paar insbesondere das Paar Ag/AgCl darstellt.

16. Vorrichtung nach einem der Ansprüche 1 bis 15, dadurch gekennzeichnet, daß der elektrochemisch zersetzbare Stoff in der Verbundstoffelektrode ein Pulver mit einer Korngröße von unter 100 µm und vorzugsweise unter 50µm ist.

17. Vorrichtung nach einem der Ansprüche 1 bis 16, dadurch gekennzeichnet, daß die Mengenanteile an elektrochemisch nicht zersetzbarer leitender Füllung und an elektrochemisch zersetzbarem Stoff, die mit dem polymeren Bindemittel verbunden sind, um die Verbundstoffelektrode zu bilden, 8 bis 45 % für die leitende Füllung und 25 bis 70 %, jeweils bezogen auf das Volumen des polymeren Bindemittels, für den elektrochemisch zersetzbaren Stoff betragen.

18. Vorrichtung nach einem der Ansprüche 1 bis 17, dadurch gekennzeichnet, daß die Verbundstoffelektrode(n), die sie umfaßt, eine Dicke von unter 100 µm und insbesondere zwischen 10 und 100 µm aufweist (aufweisen).

19. Vorrichtung nach einem der Ansprüche 1 bis 18, dadurch gekennzeichnet, daß die Verbundstoffelektrode(n), die sie umfaßt, durch Lösungsmittelbeschichtung hergestellt ist (sind).

20. Vorrichtung nach einem der Ansprüche 1 bis 19, dadurch gekennzeichnet, daß die elektrische Signalerzeugungseinrichtung zwischen den Elektroden ein intensiometrisches Signal, das heißt ein Signal mit einer erzwungenen durchschnittlichen Stromstärke, oder ein potentiometrisches Signal, das heißt ein Signal mit einer erzwungenen durchschnittlichen Spannung setzt, wobei das Signal kontinuierlich oder pulsierend und permanent oder unterbrochen sein kann, mit oder ohne zeitweise Umpolung, und mit einer Frequenz zwischen 0 und 500 kHz und insbesondere zwischen 0 und 100 kHz.

21. Vorrichtung nach Anspruch 20, dadurch gekennzeichnet, daß das zwischen den Elektroden gesetzte elektrische Signal eine durchschnittliche Spannung zwischen 0,1 und 50 Volt und insbesondere zwischen 0,5 und 20 Volt hat, so daß die Dichte des zwischen den Elektroden erzeugten durchschnittlichen Stroms einen Wert von unter 5 mA/cm² und insbesondere von unter oder gleich 1 mA/cm² aufweist.

22. Elektrodeneinheit für die Ionophorese, die eine Verbundstoffelektrode im Kontakt mit einem Behälter aufweist, der einerseits geeignet ist, einen Elektrolyten aufzunehmen, welcher einen Wirkstoff in wenigstens teilweise ionisierter Form oder in neutraler Form enthält oder ein indifferenter Elektrolyt ist, und andererseits so beschaffen ist, daß beim Kontakt mit einer Hautzone des Patienten zwischen der Verbundstoffelektrode und der Zone ein ionisches Leiterkontinuum gebildet wird, die Verbundstoffelektrode aus einer Zusammensetzung gebildet ist, die aus einem Polymerbindemittel aus 4 bis 60 % einer elektrochemisch nicht zersetzbaren pulver- oder faserförmigen leitenden Füllung und 4 bis 100 %, jeweils bezogen auf das Volumen des Bindemittels, eines durch elektrochemische Oxidation oder Reduktion zersetzbaren feinverteilten Stoffes besteht, wobei die Elektrodeneinheit dadurch gekennzeichnet ist, daß das polymere Bindemittel der Verbundstoffelektrode aus wenigstens einem Polymer auf der Basis von 1,2-Epoxy-propan und/oder 1,2-Epoxy-butan besteht und 60 bis 100 Mol.-% 1,2-Epoxy-propan und/oder 1,2-Epoxy-butan und 40 bis 0 Mol.-% eines oder mehrerer anderer mit 1,2-Epoxy-propan und/oder 1,2-Epoxy-butan copolymersierbarer Monomere enthält.

23. Elektrodeneinheit nach Anspruch 22, dadurch gekennzeichnet, daß das oder die das polymere Bindemittel der Verbundstoffelektrode bildende(n) Polymer(e) einen Molgehalt an 1,2-Epoxy-propan und/oder 1,2-Epoxy-butan von 75 bis 100 % aufweist (aufweisen).

24. Elektrodeneinheit nach Anspruch 22 oder 23, dadurch gekennzeichnet, daß das oder die das polymere Bindemittel der Verbundstoffelektrode bildende(n) Polymer(e) unter Homopolymeren von 1,2-Epoxy-propan oder 1,2-Epoxy-butan und statistischen Copolymeren, Blockcopolymeren oder Blockcopolymeren mit statistischen Gelenken aus 1,2-Epoxy-propan und 1,2-Epoxy-butan zwischen ihnen und/oder mit einem oder mehreren cyclischen Monomeren, ausgewählt aus der Gruppe, bestehend aus (i) cyclischen Oxiden mit einem Ring mit mehr als drei Gliedern und (ii) cyclischen Oxiden der Formel 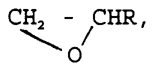 worin R einen Rest R₁ oder einen Rest -CH₂ - O - R₂ - R₁ bedeutet, wobei R₁ ein Wasserstoffatom oder einen C₃₋₁₂- und vorzugsweise C₃₋₆-Alkylrest oder einen C₂₋₁₂- und vorzugsweise C₂₋₆-Alkenylrest bezeichnet und R₂ einen Polyetherrest der Formel -(CH₂-CH₂-O)̵ₚ bedeutet, worin p eine Zahl von 0 bis 10 und vorzugsweise von 0 bis 4 bezeichnet, ausgewählt ist (sind).

25. Elektrodeneinheit nach Anspruch 24, dadurch gekennzeichnet, daß die mit dem 1,2-Epoxy-propan und/oder 1,2-Epoxy-butan copolymerisierten cyclischen Monomere unter Oxethan, Tetrahydrofuran, Dioxolan, Dioxan und cyclischen Oxiden der Formel 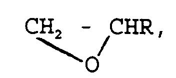 worin R einen Propyl- oder Butylrest, -CH₂OCH₃ oder -CH₂-O-CH₂-CH=CH₂ bedeutet, ausgewählt sind.

26. Elektrodeneinheit nach einem der Ansprüche 22 bis 24, dadurch gekennzeichnet, daß das oder die das polymere Bindemittel der Verbundstoffelektrode bildende(n) Polymer(e) Homopolymere von 1,2-Epoxy-propan oder 1,2-Epoxy-butan oder Copolymere dieser Oxide untereinander sind.

27. Elektrodeneinheit nach einem der Ansprüche 22 bis 26, dadurch gekennzeichnet, daß das polymere Bindemittel der Verbundstoffelektrode vernetzt ist.

28. Elektrodeneinheit nach einem der Ansprüche 22 bis 26, dadurch gekennzeichnet, daß das oder die das polymere Bindemittel der Verbundstoffelektrode bildende(n) Polymer(e) im nichtvernetzten Zustand Molekularmassen von 10.000 bis 10⁶ und insbesondere von 30.000 bis 500.000 aufweist (aufweisen).

29. Elektrodeneinheit nach einem der Ansprüche 22 bis 28, dadurch gekennzeichnet, daß die elektrochemisch nicht zersetzbare pulver- oder faserförmige leitende Füllung aus einem wenigstens aus einem Stoff wie Ruß , Graphit oder Bor gebildeten Material besteht.

30. Elektrodeneinheit nach einem der Ansprüche 22 bis 29, dadurch gekennzeichnet, daß die leitende Füllung der Verbundstoffelektrode eine pulverförmige, in Form eines Pulvers mit einer Korngröße unter 100 µm und vorzugsweise unter 50 µm vorliegende Füllung ist.

31. Elektrodeneinheit nach einem der Ansprüche 22 bis 29, dadurch gekennzeichnet, daß die leitende Füllung der Verbundstoffelektrode eine faserförmige, in Form von kurzen Fasern mit einem Durchmesser von 1 bis 15 µm und einem Verhältnis Länge : Durchmesser, ausgedrückt in derselben Längeneinheit, von 50 bis 1000 vorliegende Füllung ist.

32. Elektrodeneinheit nach einem der Ansprüche 22 bis 31, dadurch gekennzeichnet, daß der elektrochemisch zersetzbare Stoff in der Verbundstoffelektrode durch elektrochemische Oxidation zersetzbar ist, wobei die Verbundstoffelektrode eine Anode bildet und der durch elektrochemische Oxidation zersetzbare Stoff insbesondere ein Metall, ausgewählt unter Ag, Zn, Cu, Ni, Sn, Pb, Fe und Cr ist, wobei das Metall vorzugsweise Ag, Zn oder Cu ist.

33. Elektrodeneinheit nach einem der Ansprüche 22 bis 31, dadurch gekennzeichnet, daß der elektrochemisch zersetzbare Stoff in der Verbundstoffelektrode durch elektrochemische Reduktion zersetzbar ist, wobei die Verbundstoffelektrode eine Kathode bildet und der durch elektrochemische Reduktion zersetzbare Stoff insbesondere aus AgCl oder CuCl besteht.

34. Elektrodeneinheit nach einem der Ansprüche 22 bis 31, dadurch gekennzeichnet, daß der elektrochemisch zersetzbare Stoff in der Verbundstoffelektrode aus einem Paar besteht, das seinerseits aus der ionisierbaren Verbindung eines Metalls, die elektrochemisch zum Metall reduzierbare Metallionen liefert, und dem Metall besteht, wobei die Elektrode gleichermaßen eine Anode oder Kathode bildet und das Paar insbesondere das Paar Ag/AgCl darstellt.

35. Elektrodeneinheit nach einem der Ansprüche 22 bis 34, dadurch gekennzeichnet, daß der elektrochemisch zersetzbare Stoff in der Verbundstoffelektrode ein Pulver mit einer Korngröße von unter 100 µm und vorzugsweise unter 50 µm ist.

36. Elektrodeneinheit nach einem der Ansprüche 22 bis 35, dadurch gekennzeichnet, daß die Mengenanteile an elektrochemisch nicht zersetzbarer leitender Füllung und an elektrochemisch zersetzbarem Stoff, die mit dem polymeren Bindemittel verbunden sind, um die Verbundstoffelektrode zu bilden, 8 bis 45 % für die leitende Füllung und 25 bis 70 %, jeweils bezogen auf das Volumen des polymeren Bindemittels, für den elektrochemisch zersetzbaren Stoff betragen.

37. Elektrodeneinheit nach einem der Ansprüche 22 bis 36, dadurch gekennzeichnet, daß die Verbundstoffelektrode, die sie umfaßt, eine Dicke von unter 100 µm und insbesondere zwischen 10 und 100 µm aufweist.

38. Elektrodeneinheit nach einem der Ansprüche 22 bis 37, dadurch gekennzeichnet, daß die Verbundstoffelektrode, die sie umfaßt, durch Lösungsmittelbeschichtung hergestellt ist.
